# EUROPEAN PATENT APPLICATION

(11) **EP 2 853 565 A1**
(43) Date of publication of application: **01.04.2015**
(21) Application number: 13382374.0
(22) Date of filing: 27.09.2013
(51) Int. Cl.: C09B 29/036, C09B 29/08, C09B 29/42, A61K 31/54, C07D 471/00

(54) **Glutamate receptor photomodulators**

(71) Applicant: Consejo Superior De Investigaciones Científicas, 28006 Madrid (ES); Universitat Autònoma De Barcelona, 08193 Barcelona (ES); Institució Catalana de Recerca i Estudis Avançats, 08010 Barcelona (ES); Fundació Institut de Bioenginyeria de Catalunya, 08028 Barcelona (ES); Centre National de la Recherche Scientifique (CNRS), 75794 Paris Cedex 16 (FR)
(72) Inventor: Llebaria Soldevila, Amadeo, E-28006 Madrid (ES); Gorostiza Langa, Pau, E-08028 Barcelona (ES); Gomez Santacana, Xavier, E-08028 Barcelona (ES); Pittolo, Silvia, E-08028 Barcelona (ES); Giraldo Arjonilla, Jesús, 08193 Bellaterra (ES); Rovira Algans, Xavier, 34094 Montpellier cedex 5 (FR); Goudet, Cyril, 75794 Cédex 16 Paris (FR); Pin, Jean Philippe, 75794 Cédex 16 Paris (FR)
(74) Representative: ABG Patentes, S.L.

(57) **Abstract**

The present invention relates to the discovery of particular aromatic compounds of formula (1), possessing activity as modulators of metabotropic glutamate receptors (mGIuR) whose modulatory activity on the receptor may be controlled by irradiation with suitable light resulting in the optical control of receptor biological function, to the use of said compounds as a medicament, and to pharmaceutical compositions comprising said compounds of formula (1).

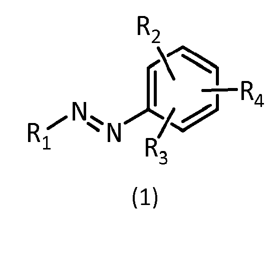

## Description

### FIELD OF THE INVENTION

The present invention relates to the discovery of particular aromatic compounds, possessing activity as modulators of metabotropic glutamate receptors (mGluR) whose modulatory activity on the receptor may be controlled by irradiation with suitable light resulting in the optical control of receptor biological function.

### BACKGROUND OF THE INVENTION

Glutamate is the principal excitatory neurotransmitter in the central nervous system acting through ionotropic and metabotropic glutamate receptors (mGluR). It also plays a major role in activating modulatory pathways through the mGluRs. The mGluRs belong to the family class III G-protein coupled receptors (GPCRs) and act either pre- or post-synaptically to modulate synaptic transmission, and are recognized as major targets for the development of new drugs of potential use in the treatment of a number of CNS diseases. Eight subtypes of mGlu receptors have been identified that define three groups according to their structure, signalling pathways and pharmacological profiles [Annu. Rev. Pharmacol. Toxicol. 2010, 50, 295-322]. Activation of group I mGluR subtypes (mGlu1, mGlu5) facilitates neuronal excitation, whereas activation of groups II (mGlu2, mGlu3) and III (mGlu4, mGlu6, mGlu7, mGlu8) mGluR subtypes inhibit synaptic transmission. Group I mGluRs are coupled to Gq/G11 proteins and activate phospholipase C, Groups II/III mGluRs are coupled to Gi/o-proteins and negatively modulate adenylate cyclase activity, thereby decreasing cAMP formation and cAMP-dependent protein kinase A [Neuropharmacology 1995, 34, 1-26].

Positive allosteric modulators (PAMs) are molecules that activate the receptor by binding to a site different from the highly conserved orthosteric binding site. On the contrary, negative allosteric modulators (NAMs) or non-competitive antagonists are compounds that deactivate receptors via alternative allosteric site(s). These allosteric modulators offer several advantages over orthosteric agonists or antagonists. First, allosteric molecules are likely to be less polar and have improved central nervous system (CNS) penetration, as they do not require glutamate functional groups necessary for the interaction with the orthosteric site. The allosteric site(s) can be less conserved and therefore allow improved mGlu receptor subtype selectivity. Allosteric modulators only act in the presence of endogenous glutamate, and, therefore, function in an activity-dependent manner and may be less prone to cause receptor desensitization [Mol. Pharmacol. 2001, 60, 881-884].

The mGluRs and specifically mGluR5 and mGluR4 are considered a potential drug target for treatment of psychiatric and neurological disorders. Treatable diseases are psychosis, epilepsy, schizophrenia, Alzheimer's disease, cognitive disorders, memory deficits, Parkinson's disease, hypoxia, ischemia, dementia caused by AIDS, migraine, depression, mood disorders and anxiety disorders. In addition, different pain syndromes can also be addressed by acting at these receptors. Other treatable indications are a drug or alcohol abuse or addiction and substance tolerance or dependence, bulimia nervosa, anorexia nervosa, gambling dependence, smoking, sex dependence, obsessive compulsive disorders or Ophelia syndrome [Neuropharmacology 2011, 60, 1017-1041; Neuropharmacology, 2013, 80, 1349-1350]. Several clinical trials (phases I, II and III) have been performed with these modulators, to treat diseases such as anxiety, fragile X syndrome, migraine, neuropathic pain, Parkinson disease or gastro-esophageal reflux, etc. [J. Clin. Psycopharmacol., 1982, 2, 129-133; J. Med. Genet., 2009, 46, 266-241; Aliment. Pharmacol. Ther., 2012, 35, 1231-1242; Mov. Disord., 2011, 26, 1243-1250; clinicaltrials.gov (NCT01019473, NCT00582673, NCT01385592, NCT01491932, NCT00857623, NCT00939094, NCT01145755, NCT00939094). In addition, mGlu5 is also linked to melanoma (PNAS 2011, 108, 15219-15224) and is also present in retinal cells (bipolar and amacrine cells in mice, all retinal cell types in chicken: Wiley Interdisciplinary Rev.: Membrane Transport and Signalling 2012, 1, 641-653; PLoS One 2010, 5(11), e15063) and therefore mGlu modulators hold therapeutic potential in these tissues.

The mGlu5 receptor protein has been localized peripherally in structures involved in nociceptive transmission and recent findings suggest that mGlu5 antagonists can be used for the treatment of different neurologic diseases. This receptor increases neuronal excitability and NMDA receptor currents in brain regions thought to be involved in anxiety, such as the amygdala [J. Comp. Neurol. 1995, 355, 455-469], leading to the hypothesis that an antagonist of mGlu5 might reduce the hyperactivity of glutamatergic transmission believed to underlie anxiety disorders. Consistent with this, mGlu5 NAMs have robust efficacy in several animal models of anxiolytic activity [Nat. Rev. Drug Discov. 2005, 4, 131-144]. Animal studies suggest that selective mGlu5 NAMs also have potential utility in the treatment of fragile X syndrome (FXS) as well as chronic pain, addictive disorders, depression, gastroesophogeal reflux, migraine, and some neurodegenerative disorders [Curr. Top. Med. Chem. 2005, 5, 897-911].

In the central nervous system, mGluR4 receptors are expressed most intensely in the cerebellar cortex, basal ganglia, sensory relay nuclei of the thalamus and hippocampus [Bradley et al., J. Comp. Neurol. 1999, 407, 33-46]. The mGluR4 is expressed primarily on presynaptic terminals, functioning as an autoreceptor or heteroceptor and activation of mGluR4 leads to decreases in transmitter release from presynaptic terminals [Corti et al., Neuroscience 2002, 110, 403-420]. Several results suggest that, among mGluR subtypes, mGluR4 is an interesting drug target for the treatment of Parkinson's disease [Conn et al., Nat. Rev. Neurosci. 2005, 6, 787-798]. In addition, behavioural studies have revealed that activation of spinal group-III mGluRs following intrathecal administration of pan group-III mGluRs agonists reduces allodynia or hyperalgesia in animal models of neuropathic or inflammatory pain while leaving acute pain perception unchanged [Chen & Pan, J. Pharmacol. Exp. Ther. 2005, 312, 120-126; Goudet et al., Pain 2008, 137, 112-124]. The antihyperalgesic effect of a selective activation of mgluR4 receptors suggests that this particular subtype may play a role in pain modulation [Wang et al., Neuroreport 2011, 22, 244-248; Goudet et al., Pain 2008, 137, 112-124].

mGluRs are implicated in the induction, expression and maintenance of both peripheral and central nociceptive sensitization, and, therefore, are considered as potential targets for novel analgesic drugs. Both iGlu receptors and several mGlu receptor subtypes have been involved in the induction and maintenance of persistent pain by producing long-lasting sensitization of the nociceptive pathways in inflammatory and neuropathic pain models. However, iGlu receptors are not considered good targets for pain treatment. Although they are effective in blocking different forms of chronic pain, the use of broad-spectrum NMDA antagonists is limited by serious adverse effects such as psychotomimetic effects and amnesia. As opposed to iGlu, mGlu receptors can be considered valid targets for the treatment of chronic pain because of their ability of modulating rather than mediating excitatory synaptic activity. Present data in the literature indicate that blocking group I mGluRs or activating group II or III mGluRs produce antinociceptive effects [Curr. Opin. Pharmacol. 2012, 12, 28-34].

Document WO 99/02497 A2 discloses 2-arylazopyridine derivatives having non-competitive antagonistic activity on the mGlu5 receptor. However, this document does not show any light-dependent modulation of the activity of the compounds disclosed therein.

Another singular feature of the mGluRs modulators refers to the multiple site of action on receptors localized in different tissues or brain and nerve regions, resulting in multiple responses that cannot be controlled by means of the classical drug treatments. Although local administration of drugs acting on mGluRs has been used to address the role for different mGluRs in pain [Carlton et al., Neuroscience Lett. 2011, 500, 197-201], its practical use is difficult and some challenges remain unsolved, including the control of the drug action site, the time course of drug effect, and the localization of drug action to peripheral nervous system or central nervous system.

A possible improvement would be the introduction of an optical control system of mGluR activity. The optical control of protein function is possible in different ways. Specific interest have non-invasive methods to manipulate neuronal activity, optogenetic [Science 2009, 326, 395-399], and photostimulation [Nat. Methods 2007, 4, 943-950] are currently being applied to a variety of animal models, but optogenetic gene targeting (e.g. using viral vectors) needs to be thoroughly tested for human applications. It would then be desirable to have a direct molecular approach that can operate reversibly in native (non-mutated) proteins. One possibility is to use photochromic ligands (PCLs) of receptor proteins [Science 2008, 322, 395-399], which are molecules that bind to that protein acting as agonists, antagonists or modulators of the protein activity with a light-dependent affinity. These molecules do not require genetic manipulation in human subjects and can be developed following well-established procedures for discovery and validation of drugs. For example, PCLs have been developed for kainate receptors or for nicotinic acetylcholine receptors. These PCLs consist in modification of the orthosteric ligand structure by covalently attaching to it a photoisomerizable group (such as an azobenzene) that it is capable of altering drug affinity [Mol. Biosyst. 2007, 3, 686-704]. However, the resulting PCL compounds are orthosteric ligands and have the selectivity, penetration and desensitization general problems associated to this type of ligands. In addition, the mechanism of action of the azobenzene photoisomerizable group is to alter affinity by inducing with light a compound configuration in which the ligand is less exposed to the binding site. In practice this strategy is limited by the partial conversion cis/trans of the azobenzene and the poor affinity of the orthosteric ligands. This results in a very narrow concentration range in which the PCLs can be effectively photoswitched (10-100 microM in the case of Volgraf et al. [J. Am. Chem. Soc. 2007, 129, 260-261].

A successful strategy for improving signalling pathway selectivity has been the development of designer GPCRs' that are activated by light. These receptors can be targeted to genetically defined cell populations but require exogenous expression and functional substitution of endogenous receptors, and are insensitive to endogenous ligands [Exp. Physiol. 2011, 96, 51-56].

One possibility to combine the benefits of optical and pharmacological manipulation of endogenous receptors would be developing receptor type-selective ligands that can be directly regulated with light in order to pattern their activation with high time-space precision. For instance, control of protein function has indeed been demonstrated in several ion channels [ACS Chem. Neurosci. 2011, 2, 536-543] and receptor-channels [J. Am. Chem. Soc. 2007, 129, 260-261] but not in GPCRs, despite of their importance as drug targets.

The photoisomerization induces a switch between active and inactive configurations of the PCL, which can result in the activation and deactivation of the receptor. The therapeutic applications of PCLs have been hampered because an active compound would require a nanomolar receptor affinity, high receptor selectivity and a wide concentration range for effective light switching. The use of PCLs acting at allosteric sites would be therefore advantageous in two directions (stronger affinity and better receptor selectivity) but no allosteric PCLs have been described to date. However, a clear-cut activation-deactivation response over a wide range of concentrations appears difficult to be obtained based upon light-dependent agonist or antagonist affinity, because of the partial trans to cis conversion ratio or low differential affinity of the isomers. Another factor that reduces the efficacy of PCLs is due to the fact that light induces a change from an active conformer (agonist or antagonist) characterized by protein binding to an inactive one with lower affinity for the receptor. To increase the light induced response to useful levels, it would be desirable to obtain molecules having two high affinity binding configurations inducing opposite receptor response, shifting from inactivation to activation and vice versa upon suitable irradiation, this inducing changes from antagonism to agonism with a single molecule, that would have a more pronounced receptor effect.

The present invention demonstrates that light-regulated ligands can also be designed for deep and tight allosteric binding pockets of GPCRs, and describes high-affinity photochromic allosteric modulators of mGlu5 that inhibit the receptor responses under specific light frequencies and enhance them under a different range of light frequencies, thus overcoming the problems of the modulators known in the prior art.

Surprisingly, we have found non-competitive PCL compounds of mGlu5 capable of inducing the light-dependent modulation (inhibition or potentiation of this receptor) at concentrations ranging from 0.1 nM to 25 µM. Said non-competitive PCL compounds of mGlu5, act as a non-competitive antagonists of the mGlu5 response at some wavelengths and as potentiators of said mGlu5 response at some others. These molecules induce a switch in mGlu5 response in cells, reducing, maintaining or potentiating it, by the proper selection of light frequencies.

The identified photochromic allosteric modulators of mGlu5 also display the novelty of changing its pharmacodynamic character with light. An example of this behaviour can be compound **1c**, whose trans isomer acts as a potent non-competitive antagonist that switches to the cis configuration, resulting in a potentiation of the response of the receptor, providing a modulation capable of generating stimulus-bias in orthosteric ligand signalling in a light-regulated manner. This property offers exciting opportunities in the photoregulation of receptor-selective and signalling-pathway-selective therapies [Trends Pharmacol. Sci. 2007, 28, 382-389]. In particular, it could lead to 'single-button' regulation of inhibition and potentiation, which may be useful in pathological states characterized by unbalanced signalling or in which homeostatic mechanisms have failed. The spatiotemporal regulation of drug action by GPCR optopharmacology has the potential to tackle other therapeutic challenges like the personalized application of temporally precise patterns of drug action, or the reduction of side effects due to receptor expression in non-targeted tissues, through the spatial localization of drug action.

US 2007/0128662 A2 describes synthetic regulators of protein function (including agonists of the glutamate receptors) which comprise a photoisomerizable group and whose agonistic activity is susceptible of being photomodulated. The reference describes a long list of photoisomerizable group comprising also azobenzene groups.

In spite of the teaching of US 2007/0128662 A2, the inventors have now found that not all azobenzene molecules with affinity for a protein and specifically for a GPCR glutamate receptor such as mGlu5 are susceptible of being photomodulated. This is illustrated in the comparative examples of the present invention showing that the antagonistic activity on the mGlu5 receptor of compound SIB-1757, a classic mGlu5 non-competitive antagonist comprising an azobenzene group cannot be modulated by light exposure. Moreover, it has also been observed that not all double bonds (C=C or N=N) in an allosteric ligand having a specific geometry, either *cis* or *trans,* define a similar pharmacological profile. Also, substitution of a C=C bond by a N=N bond in a protein ligand does not allow an obvious prediction of its pharmacological profile, its affinity and its ability of the photocontrol of the protein activity, as it can be seen in the comparative examples provided in the present invention. The prior art fails to provide any guidance as to the chemical structural features of a molecule that is required to obtain a photoswithchable dual antagonist-enhancer.

The inventors have now surprisingly discovered the compounds of formula (1), defined below, which have mGlu5 modulatory activity that may be controlled by irradiation with suitable light resulting in the optical control of receptor biological function.

### SUMMARY OF THE INVENTION

In a first aspect, the present invention is directed to a compound of formula (1): 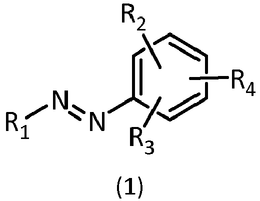 wherein
R₁ is selected from 2-pyridyl and 3-pyridyl, optionally substituted at the carbon atoms with one, two or three substituents independently selected from the group consisting of -halogen, -OH, -OR₅, -OCF₃, -SR₅, -NR₅R₆, -NHCOR₅, -COOR₅, -OCOR₅, -NO₂, -COR₅,-CONR₅R₆, -SO₂NH₂, (C₁-C₆) alkyl and (C₆-C₁₀) aryl;
R₂ and R₃ are independently selected from -H, halogen, -OR₅, -OCF₃, -SR₅, -NR₅R₆, -NHCOR₅, -COOR₅, -OCOR₅, -NO₂, -CN, -COR₅, -CONR₅R₆, -SO₂NH₂, -NHSO₂R₅, (C₁-C₆) alkyl and (C₆-C₁₀) aryl;
R₄ is selected from -NR₇CO-R₈, -CONR₇R₉, -NHSO₂-R₉, -S-R₉, -O-R₉, -NR₇CONH-R₁₀ and-N-phthalimidyl group;
R₅ and R₆ are independently selected from H, (C₁-C₆) alkyl and (C₆-C₁₀) aryl;
R₇ is selected from H, -CO-(C₁-C₅) alkyl, -CO-(C₆-C₁₀) aryl, (C₄-C₆) alkyl, (C₆-C₁₀) aryl and heterocyclyl; wherein the heterocyclyl group is linked to the -CON- or -NCO moieties by a ring carbon atom;
R₈ is selected from (C₃-C₈) alkyl; (C₃-C₈) cycloalkyl; (C₆-C₁₀) aryl and heterocyclyl; wherein the heterocyclyl group is linked to the -NR₇CO- moiety by a ring carbon atom;
R₉ is selected from (C₁-C₈) alkyl, (C₃-C₈) cycloalkyl, (C₆-C₁₀) aryl and heterocyclyl; wherein the heterocyclyl group is linked to the -CON-, -NHSO₂-, -S-, or -O- moieties by a ring carbon atom
R₁₀ is selected from (C₁-C₈) alkyl, (C₃-C₈) cycloalkyl and (C₆-C₁₀) aryl;
wherein the alkyl, aryl, cycloalkyl and heterocyclyl moieties in R₂-R₃ and R₅-R₁₀ defined above are optionally substituted, with one, two or three substituents independently selected from the group consisting of -halogen, -OH, -OR₅, -OCF₃, -SR₅, -NR₅R₆,
-NHCOR₅, -COOR₅, -OCOR₅, -NO₂, -COR₅, -CONR₅R₆, -SO₂NH₂, (C₁-C₆) alkyl and (C₆-C₁₀) aryl;
R₄ is in *meta* or *para* position in respect to the R₁-N=N- moiety; and provided that the compound of formula (1) contains from 2 to 4 ring systems, only 1 - N=N- group and that the compound of formula (1) is not 2-amino-N-[4-[2-(3-pyridinyl)diazenyl]phenyl]benzamide;
or a pharmaceutically acceptable salt, stereoisomer or solvate thereof.

In the second aspect, the present invention is directed to a compound of formula (1) as defined in the first aspect, or a pharmaceutically acceptable salt stereoisomer or solvate thereof, for use as a medicament.

In a third aspect, the present invention is directed to a compound of formula (1) as defined in the first aspect, or a pharmaceutically acceptable salt, stereoisomer or solvate thereof, for use in the treatment and/or prevention of a disease or condition selected from the group consisting of psychosis, epilepsy, schizophrenia, Alzheimer's disease, cognitive disorders, memory deficits, Parkinson's disease, hypoxia, ischemia, dementia caused by AIDS, migraine, depression, mood disorders, anxiety disorders, fragile X syndrome, pain syndromes (such as neuropathic pain and chronic pain), addictive disorders (such as drug or alcohol abuse or addiction and substance tolerance or dependence, and drug withdrawal), bulimia nervosa, anorexia nervosa, gambling dependence, smoking, sex dependence, obsessive compulsive disorders, stomach diseases (such as gastroesophageal reflux), and Ophelia syndrome.

In a fourth aspect, the present invention is directed to a pharmaceutical composition comprising a compound of formula (1) as defined in the first aspect, or a pharmaceutically acceptable salt, stereoisomer or solvate thereof, and a pharmaceutically acceptable excipient.

### DESCRIPTION OF THE FIGURES

Figure 1 shows the spectrum of absorption in UV-VIS of a solution of SIB-1757 in DMSO a) in the relaxed state (without irradiation); and b) after irradiation at 380 nm. The x-axis represents the light wavelengths (nm) and the y-axis the absorption of the sample.

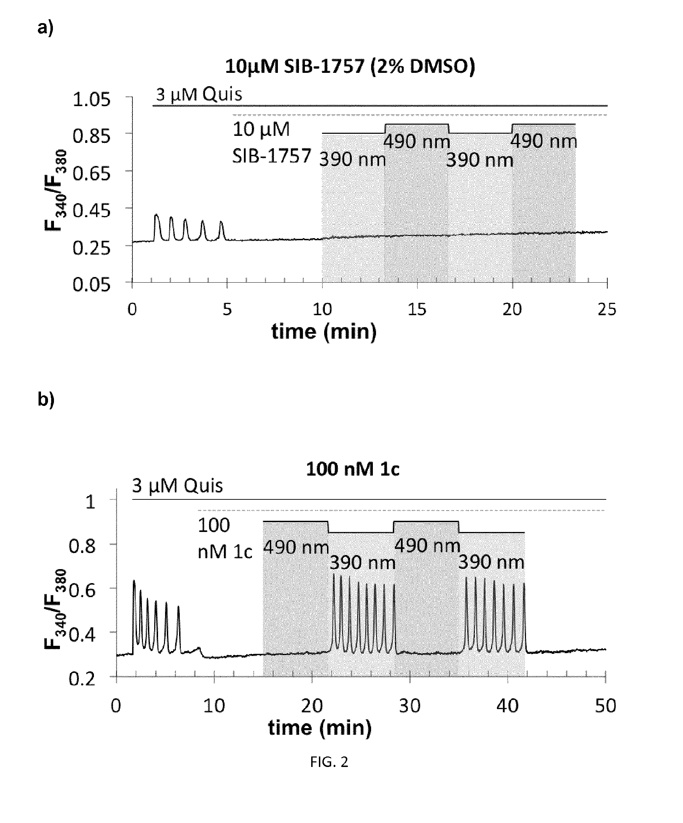

Figure 2 shows the intracellular calcium oscillation (as the ratio of fluorescence intensities recorded at 510 nm after excitation at 340 nm and 380 nm, F₃₄₀/F₃₈₀, with respect to the time) on HEK tsA201 cells transiently expressing the rat metabotrobic glutamate receptor 5 (mGlu5) after activation of mGlu5 by addition of 3 µM quisqualate, followed by addition of SIB-1757 (figure 2a) or compound **1c** (figure 2b). The cis-isoforms of the two molecules were obtained by illuminating at 380 or 390 nm, respectively, represented as a clearer box, and the *trans*-configurations were restored under 500 and 490 nm light, respectively, represented as a darker box.
Figure 3 shows dose (concentration in nM) - response (oscillations/minute) curves in the dark and under 490 nm and 390 nm illumination from calcium responses to quisqualate and to compound **1c** at different concentrations.
Figure 4 shows the intracellular calcium oscillation (F₃₄₀/F₃₈₀) with respect to the time (min) on HEK tsA201 cells transiently expressing the rat metabotrobic glutamate receptor 5 (mGlu5) after activation of mGlu5 by addition of 3 µM quisqualate, followed by addition of compound *E*-(47) (figure 4a) or compound Z-(47) (figure 4b).

### DESCRIPTION OF THE INVENTION

### Definitions

The term "alkyl" as employed herein alone or as part of another group designates both straight- and branched-chain saturated hydrocarbons containing the number of carbon atoms indicated along the invention and attached to the rest of the molecules through a single bond. Examples of alkyl groups are methyl, ethyl, n-propyl, isopropyl, n-butyl, isobutyl, tert-butyl, pentyl, hexyl, isohexyl, heptyl, 4,4-dimethylpentyl, octyl, 2,2,4-trimethylpentyl, nonyl, decyl, and the various branched-chain isomers thereof. The alkyl groups can be optionally substituted by one or more substituents selected independently from -halogen, -OR₅, -OCF₃, -SR₅, -NR₅R₆, -NHCOR₅, -COOR₅, -OCOR₅, - NO₂, -COR₅, -CONR₅R₆, -SO₂NH₂, (C₁-C₆)alkyl and (C₆-C₁₀) aryl, wherein, R₅ and R₆ are independently selected from H, optionally substituted (C₁-C₆)alkyl and optionally substituted (C₆-C₁₀)aryl as defined above.

The term "cycloalkyl" as employed herein alone or as part of another group refers to saturated or partially saturated monocyclic hydrocarbon groups, and containing a total of 3 to 10 carbon atoms, preferably 3 to 8 carbons forming part of the ring system. Examples of cycloalkyl groups are cyclopropyl, cyclobutyl, cyclopentyl, cyclohexyl, cycloheptyl, cyclooctyl, cyclodecyl and adamantyl. The cycloalkyl groups can be optionally substituted by one to three substituents, selected independently from - halogen, -OR₅, -OCF₃, -SR₅, -NR₅R₆, -NHCOR₅, -COOR₅, -OCOR₅, -NO₂, -COR₅, -CONR₅R₆,-SO₂NH₂, (C₁-C₆)alkyl and (C₆-C₁₀) aryl, wherein, R₅ and R₆ are independently selected from H, optionally substituted (C₁-C₆)alkyl and optionally substituted (C₆-C₁₀)aryl.

The term "aryl" as employed herein alone or as part of another group refers to monocyclic or bicyclic aromatic hydrocarbon groups containing 6 to 10 carbons in the ring portion, such as phenyl or naphthyl (including 1-naphthyl and 2-naphthyl), having optionally one or more substituents, preferably from one to three, selected independently from -halogen, -OH, -OR₅, -OCF₃, -SR₅, -NH₂, -NR₅R₆, -NHCOR₅, -COOR₅,-OCOR₅, -NO₂, -COR₅, -CONR₅R₆, -SO₂NH₂, (C₁-C₆)alkyl and (C₆-C₁₀) aryl, wherein, R₅ and R₆ are independently selected from H, optionally substituted (C₁-C₆)alkyl and optionally substituted (C₆-C₁₀)aryl.

The term "heterocyclyl" as employed herein alone or as part of another group refers to a stable radical of 4 to 8 members that consists in carbon atoms and from one to three heteroatoms selected from the group consisting in N, O and S, preferably a ring of 5 or 6 members with one or more heteroatoms. For this invention, the heterocycle can be a system of monocyclic or bicyclic rings, which can include condensed ring systems, and the heterocyclyl radical can be saturated, partially or fully unsaturated or be aromatic and it is linked to the compound by a ring carbon atom. Examples of such heterocycles may be, not limited to thiazolyl, benzimidazolyl, benzothiazolyl, furanyl, isothiazolyl, indolyl, pyridyl, piperidinyl, piperazinyl, thiadiazolyl, tetrahydrofuranyl, coumarine, morpholinyl, pyrrolyl, pyrazolyl, imidazolyle, pyrrolidinyl, tetrahydrofuranyl, etc. The heterocycle may be optionally substituted with one or more substituents, preferably from one to three, selected independently from -halogen, -OH, -OR₅, -OCF₃, -SR₅, -NH₂, -NR₅R₆, -NHCOR₅, -COOR₅, -OCOR₅, -NO₂, -COR₅, -CONR₅R₆, -SO₂NH₂, (C₁-C₆)alkyl and (C₆-C₁₀) aryl.

For this invention, 2- and 3- pyridine cannot have quaternized the nitrogen group.

The term "halogen" as used herein alone or as part of another group refers to chlorine, bromine, fluorine, and iodine.

The term "hydroxyl" as used herein alone or as part of another group refers to -OH.

The term "ring system" is to be understood as a single ring or two or more rings that are fused across a bond between two atoms, i.e., naphthalene, across a sequence of atoms (bridgehead), i.e. norbornane, or at a single atom (spirocyclic), i.e. spiro[3.4]octane. Two rings connected by a bond, i.e., biphenyl, are not considered a single ring system but are considered as two separate ring systems.

As used herein, the term "pharmaceutically acceptable salt" embraces salts with a pharmaceutically acceptable acid or base, which are synthesized from the parent compound which contains an acidic moiety by addition of a pharmaceutically acceptable base, or which are synthesized from the parent compound which contains a basic moiety by addition of a pharmaceutically acceptable acid. Pharmaceutically acceptable acids include both inorganic acids, for example, hydrochloric (HCl), sulfuric (H₂SO₄), phosphoric (H₃PO₄), diphosphoric (H₄P₂O₇), hydrobromic (HBr), hydroiodic (HI), and nitric acid (HNO₃) and organic acids, for example, citric, fumaric, maleic, malic, mandelic, ascorbic, oxalic, succinic, tartaric, benzoic, acetic (AcOH), methanesulfonic, ethanesulfonic, benzenesulfonic, or *p*-toluenesulfonic acid. Pharmaceutically acceptable bases include alkali metal (e.g., sodium or potassium) and alkali earth metal (e.g., calcium or magnesium) hydroxides and organic bases, such as alkyl amines, arylalkyl amines, and heterocyclic amines.

All stereoisomers of the compounds of this invention are contemplated either alone or as mixtures thereof. Stereoisomers refer to compounds having stereogenic centres, e.g. enantiomers, diastereomers, meso and racemic forms, and to compounds having different substituents on the atoms linked to form a multiple bond, such as a double or triple bond (e.g. -HC=CH-, -C≡C-, -N=N-, etc.), i.e. *cis (Z)* and *trans* (*E*) isomers. When diastereomeric or enantiomeric products are prepared, they can be separated by conventional methods, for example, chromatographic or functional crystallization. When *cis* and *trans* isomers of the compounds of formula (1) according to the present invention are prepared they can be reversibly interconverted from one isomer to the other isomer by irradiation at the appropriate wavelength, i.e., to obtain the *trans* isomer from the *cis* isomer, a wavelength between 310 and 500 nm may be used, and to obtain the *cis* isomer from the *trans* isomer irradiation at a wavelength between 425 and 600 nm may be used.

### Compounds of the invention

In a first aspect, the present invention is directed to a compound of formula (1) as defined above.

In one embodiment, the present invention provides a compound of formula (1), wherein R₁ is unsubstituted 2-pyridyl.

In another embodiment, the present invention provides a compound of formula (1) wherein R₂ and R₃ are independently selected from -H, -OR₅, and -CN; preferably wherein R₂ and R₃ are independently selected from H and -OR₅; even more preferably, wherein R₂ and R₃ are independently selected from H, unsubstituted -O-(C₁-C₆) alkyl; and still more preferably, wherein R₂ and R₃ are independently selected from H, -OMe, and -OEt. In a more preferred embodiment, one of R₂ and R₃ is H; even more preferably, wherein R₂ is H and R₃ is in *meta* position in respect to the R₁-N=N- moiety.

In another embodiment, the present invention provides a compound of formula (1) as previously defined (i.e., wherein R₂ and R₃ are independently selected from -H, -OR₅, and -CN), wherein one of R₂ and R₃ is -OR₅ and wherein R₅ is selected from unsubstituted (C₁-C₆) alkyl; preferably, wherein one of R₂ and R₃ is H; even more preferably, wherein R₂ is H and R₃ is in *meta* position in respect to the R₁-N=N- moiety.

In another embodiment, the present invention provides a compound of formula (1) as defined above, wherein R₄ is selected from -NR₇CO-R₈, -CONR₇R₉, and -NR₇CONH-R₁₀ and N-phthalimidyl.

In a preferred embodiment R₇ is selected from H and optionally substituted CO-(C₆-C₁₀) aryl; even more preferably R₇ is H or -CO-phenyl optionally substituted with CN; still more preferably R₇ is H.

In another preferred embodiment, R₈ is selected from the group consisting of unsubstituted (C₃-C₈) alkyl; unsubstituted (C₃-C₈) cycloalkyl; phenyl optionally substituted with 1, 2 or 3 substituents independently selected from -CO-phenyl, halogen, and -CN; pyridyl; thiazolyl; preferably, R₈ is selected from unsubstituted (C₃-C₅) alkyl; unsubstituted (C₅-C₇) cycloalkyl; phenyl substituted with 1 or 2 substituents independently selected from halogen, preferably Cl; and pyridyl.

In another preferred embodiment, R₉ is selected from the group consisting of unsubstituted (C₃-C₈) cycloalkyl; phenyl optionally substituted with 1, 2 or 3 substituents independently selected from -CO-phenyl, halogen, and -CN; pyridyl and thiazolyl; preferably, R₉ is pyridyl; even more preferably, R₉ is 2-pyridyl.

In another preferred embodiment, R₁₀ is phenyl optionally substituted with 1 or 2 substituents selected from halogen, preferably F.

In a preferred embodiment, in the compound of formula (1), R₄ is -NR₇CO-R₈, wherein R₇ is selected from H and optionally substituted CO-(C₆-C₁₀) aryl; more preferably wherein R₇ is selected from H and optionally substituted CO-(C₆-C₁₀) aryl; even more preferably wherein R₇ is H or -CO-phenyl optionally substituted with CN; still more preferably wherein R₇ is H.

In another embodiment, the present invention provides a compound of formula (1) as defined above, wherein R₄ is -NR₇CO-R₈ and wherein R₈ is selected from unsubstituted (C₃-C₈) alkyl; unsubstituted (C₃-C₈) cycloalkyl; phenyl optionally substituted with 1, 2 or 3 substituents independently selected from -CO-phenyl, halogen, and -CN; pyridyl; thiazolyl. Preferably, wherein R₈ is selected from unsubstituted (C₃-C₅) alkyl; unsubstituted (C₅-C₇) cycloalkyl; phenyl substituted with 1 or 2 substituents independently selected from halogen, preferably Cl; and pyridyl.

In a particular embodiment, the present invention provides a compound of formula (1) as defined above, wherein R₄ is -NR₇CO-R₈; wherein R₇ is selected from H and optionally substituted CO-(C₆-C₁₀) aryl and R₈ is selected from unsubstituted (C₃-C₈) alkyl; unsubstituted (C₃-C₈) cycloalkyl, phenyl optionally substituted with 1, 2 or 3 substituents independently selected from -CO-phenyl, halogen, and -CN; more preferably wherein R₇ is H or -CO-phenyl optionally substituted with CN, and R₈ is selected from unsubstituted (C₃-C₅) alkyl; unsubstituted (C₅-C₇) cycloalkyl; phenyl substituted with 1 or 2 substituents independently selected from halogen (preferably Cl) and pyridyl; still more preferably wherein R₇ is H and R₈ is selected from unsubstituted (C₃-C₅) alkyl; unsubstituted (C₅-C₇) cycloalkyl; phenyl substituted with 1 or 2 substituents independently selected from halogen (preferably Cl) and pyridyl.

In another embodiment, the present invention provides a compound of formula (1) as defined above, wherein R₄ is -CONR₇-R₉, wherein R₉ is selected from unsubstituted (C₃-C₈) cycloalkyl; phenyl optionally substituted with 1, 2 or 3 substituents independently selected from -CO-phenyl, halogen, and -CN; pyridyl and thiazolyl; preferably, wherein R₉ is selected from pyridyl; even more preferably, wherein R₉ is selected from 2-pyridyl. Preferably, R₇ is H.

In a particular embodiment, the present invention provides a compound of formula (1) as defined above, wherein R₄ is -CONR₇-R₉; wherein R₇ is selected from H and optionally substituted CO-(C₆-C₁₀) aryl and R₉ is selected from (C₃-C₈) cycloalkyl, phenyl optionally substituted with 1, 2 or 3 substituents independently selected from -CO-phenyl, halogen, and -CN; more preferably wherein R₇ is H or -CO-phenyl optionally substituted with CN, and R₉ is pyridyl; still more preferably wherein R₇ is H and R₉ is pyridyl, preferably 2-pyridyl.

In another embodiment, the present invention provides a compound of formula (1) as defined above, wherein R₄ is -NR₇CONH-R₁₀ and wherein R₁₀ is selected from (C₆-C₁₀) aryl optionally substituted with one, two or three substituents independently selected from halogen, CN and OH. Preferably, R₁₀ is selected from phenyl substituted with 1 or 2 substituents independently selected from halogen, preferably F. Preferably, R₇ is H.

In a particular embodiment, the present invention provides a compound of formula (1) as defined above, wherein R₄ is -NR₇CONH-R₁₀; wherein R₇ is selected from H and optionally substituted CO-(C₆-C₁₀) aryl and R₁₀ is selected from (C₆-C₁₀) aryl optionally substituted with one, two or three substituents independently selected from halogen; more preferably wherein R₇ is H and R₁₀ is selected from phenyl substituted with 1 or 2 substituents independently selected from halogen, preferably F.

In another embodiment, the present invention provides a compound of formula (1) as defined above, wherein R₄ is in *para* position in respect to the R₁-N=N- moiety.

In another embodiment, the present invention provides a compound of formula (1) selected from the group consisting of:
- (E)-4-benzoyl-*N*-(2-methoxy-4-(pyridin-2-yldiazenyl)phenyl)benzamide;
- (Z)-4-benzoyl-*N*-(2-methoxy-4-(pyridin-2-yldiazenyl)phenyl)benzamide;
- (E)-2,4-dichloro-*N*-(2-methoxy-4-(pyridin-2-yldiazenyl)phenyl)benzamide;
- (Z)-2,4-dichloro-*N*-(2-methoxy-4-(pyridin-2-yldiazenyl)phenyl)benzamide;
- (E)-2-chloro-*N*-(2-methoxy-4-(pyridin-2-yldiazenyl)phenyl)benzamide;
- (Z)-2-chloro-*N*-(2-methoxy-4-(pyridin-2-yldiazenyl)phenyl)benzamide;
- (E)-*N*-(2-methoxy-4-(pyridin-2-yldiazenyl)phenyl)picolinamide;
- (*Z*)-*N*-(2-methoxy-4-(pyridin-2-yldiazenyl)phenyl)picolinamide;
- (E)-*N*-(2-methoxy-4-(pyridin-2-yldiazenyl)phenyl)nicotinamide;
- (Z)-*N*-(2-methoxy-4-(pyridin-2-yldiazenyl)phenyl)nicotinamide;
- (E)-*N*-(2-methoxy-4-(pyridin-2-yldiazenyl)phenyl)isonicotinamide;
- (Z)-*N*-(2-methoxy-4-(pyridin-2-yldiazenyl)phenyl)isonicotinamide;
- (E)-*N*-(2-cyano-4-(pyridin-2-yldiazenyl)phenyl)picolinamide;
- (Z)-*N*-(2-cyano-4-(pyridin-2-yldiazenyl)phenyl)picolinamide;
- (E)-*N*-(4-(pyridin-2-yldiazenyl)phenyl)picolinamide;
- (Z)-*N*-(4-(pyridin-2-yldiazenyl)phenyl)picolinamide;
- (E)-*N*-(3-methoxy-4-(pyridin-2-yldiazenyl)phenyl)picolinamide;
- (E)-2-(2-methoxy-4-(pyridin-2-yldiazenyl)phenyl)isoindoline-1,3-dione;
- (Z)-2-(2-methoxy-4-(pyridin-2-yldiazenyl)phenyl)isoindoline-1,3-dione;
- (E)-*N*-benzoyl-N-(2-methoxy-4-(pyridin-2-yldiazenyl)phenyl)benzamide;
- (Z)-*N*-benzoyl-N-(2-methoxy-4-(pyridin-2-yldiazenyl)phenyl)benzamide;
- (E)-*N*-(2-methoxy-4-(pyridin-2-yldiazenyl)phenyl)benzamide;
- (Z)-*N*-(2-methoxy-4-(pyridin-2-yldiazenyl)phenyl)benzamide;
- (E)-*N*-(2-methoxy-4-(pyridin-2-yldiazenyl)phenyl)isobutyramide;
- (Z)-*N*-(2-methoxy-4-(pyridin-2-yldiazenyl)phenyl)isobutyramide;
- (E)-*N*-(2-methoxy-4-(pyridin-2-yldiazenyl)phenyl)cyclohexanecarboxamide;
- (Z)-*N*-(2-methoxy-4-(pyridin-2-yldiazenyl)phenyl)cyclohexanecarboxamide;
- (E)-1-(2-fluorophenyl)-3-(2-methoxy-4-(pyridin-2-yldiazenyl)phenyl)urea;
- (Z)-1-(2-fluorophenyl)-3-(2-methoxy-4-(pyridin-2-yldiazenyl)phenyl)urea;
- (E)-2-fluoro-N-(2-methoxy-4-(pyridin-2-yldiazenyl)phenyl)benzamide;
- (Z)-2-fluoro-N-(2-methoxy-4-(pyridin-2-yldiazenyl)phenyl)benzamide;
- (E)-2-cyano-N-(2-cyanobenzoyl)-N-(2-methoxy-4-(pyridin-2-yldiazenyl)phenyl)benzamide;
- (Z)-2-cyano-N-(2-cyanobenzoyl)-N-(2-methoxy-4-(pyridin-2-yldiazenyl)phenyl)benzamide;
- (E)-*N*-(2-methoxy-4-(pyridin-2-yldiazenyl)phenyl)thiazole-4-carboxamide;
- (Z)-*N*-(2-methoxy-4-(pyridin-2-yldiazenyl)phenyl)thiazole-4-carboxamide;
- (E)-*N*-(pyridin-2-yl)-4-(pyridin-2-yldiazenyl)benzamide;
- (Z)-*N*-(pyridin-2-yl)-4-(pyridin-2-yldiazenyl)benzamide;
- (E)-*N*-(3-(pyridin-2-yldiazenyl)phenyl)picolinamide; and
- (Z)-*N*-(3-(pyridin-2-yldiazenyl)phenyl)picolinamide;
or a pharmaceutically acceptable salt, stereoisomer or solvate thereof.

In another preferred embodiment, the present invention provides a compound of formula (1) selected from the group consisting of:
- (E)-2-chloro-N-(2-methoxy-4-(pyridin-2-yldiazenyl)phenyl)benzamide;
- (E)-*N*-(2-methoxy-4-(pyridin-2-yldiazenyl)phenyl)picolinamide;
- (E)-*N*-(2-methoxy-4-(pyridin-2-yldiazenyl)phenyl)nicotinamide;
- (E)-*N*-(2-methoxy-4-(pyridin-2-yldiazenyl)phenyl)isobutyramide;
- (E)-*N*-(2-methoxy-4-(pyridin-2-yldiazenyl)phenyl)cyclohexanecarboxamide
- (E)-2-fluoro-N-(2-methoxy-4-(pyridin-2-yldiazenyl)phenyl)benzamide; and
- (E)-*N*-(2-Methoxy-4-(pyridin-2-yldiazenyl)phenyl)thiazole-4-carboxamide;
or a pharmaceutically acceptable salt, stereoisomer or solvate thereof.

### Synthesis of compounds of formula (1)

The compounds of formula (1) of the invention can be prepared as shown in the following reaction schemes and description thereof.

If not said specifically, radicals R₁-R₁₀ are as defined above for the compounds of formula (1).

In the following synthetic schemes, the compounds according to the present invention and their synthetic intermediates and precursors are depicted with the corresponding R₄ group in *para* position to the corresponding R₁-N=N moiety, but they do also encompass the compounds of the present invention and their synthetic intermediates and precursors wherein the corresponding R₄ is in the meta position with respect to the corresponding R₁-N=N moiety using the suitable meta starting compounds, except in the case of schemes 2, 5 and 8 where the corresponding final compounds with R₄ group in meta position cannot be obtained using the methodologies depicted therein.

In a first approach, compounds of formula (6), i.e. compounds of formula (1) wherein R₄ is -NR₇CO-R₈, can be prepared following the synthetic pathway depicted in Scheme 1 comprising the optional derivatisation of an aniline (step a), formation of amide or imide (4), from an aniline of formula (2) (derivatized or not) and a compound of formula (3) (step b), followed by reduction of nitro group of the compound of formula (4) to give an amino derivative of formula (5) (step c), and azo-bond formation to give the compounds of formula (6), (step d).

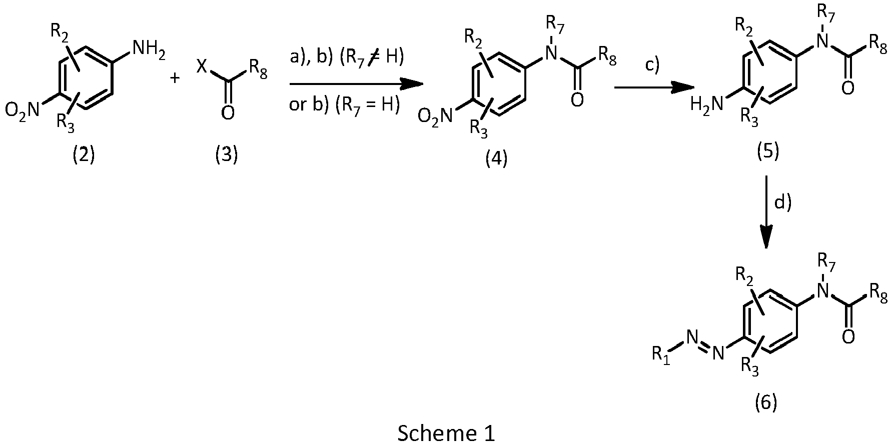

Thus, in step b) compounds of general formula (4) may be prepared by reacting aniline of general formula (2) with a compound (3) suitable for the synthesis of amide (4), such as those selected from carboxylic acid, acyl chloride and carboxylic anhydride. The reaction may be carried out in one pot in a solvent such as dichloromethane (DCM), 1,2-dichloroethane (DCE), ethyl acetate (EtOAc), tetrahydrofuran (THF), 1,4-dioxane, dimethyl formamide (DMF), AcOH, diethylether (Et₂O), toluene, xylene, etc., optionally in the presence of one or more coupling agents such as N-(3-dimethylaminopropyl)-N'-ethylcarbodiimide (EDC), N,N'-diisopropylcarbodiimide (DIC), N,N'-dicyclohexylcarbodiimide (DCC), bis(trimethylsilyl)carbodiimide N,N'-di-tert-butylcarbodiimide, 1-hydroxybenzotriazole (HOBt), O-(benzotriazol-1-yl)-N,N,N',N'-tetramethyluronium hexafluorophosphate (HBTU), O-(benzotriazol-1-yl)-N,N,N',N'-tetramethyluronium tetrafluoroborate (TBTU), O-(7-azabenzotriazol-1-yl)-N,N,N',N'-tetramethyluronium hexafluorophosphate (HATU), N-hydroxysuccinimide (HOSu), 4-nitrophenyl chloroformate, 4-nitrophenyl trifluoroacetate, carbonyl dimiadazole (CDI), bis(4-nitrophenyl) carbonate, N,N'-disuccinimidyl carbonate (DSC), etc. in a presence of a base such as triethylamine (TEA), diisopropylethylamine (DIPEA), Hünig's base, sodium hydride (NaH), potassium carbonate (K₂CO₃), alumina, etc. in a standard reactor or flask between 0ºC and 150ºC. Optionally catalysts such as N,N-dimethylpyridin-4-amine (DMAP) may be used.

Alternatively, when R₇ ≠ H compounds of general formula (4), the compounds of general formula (2) can be derivatized to an *N*-alkyl-aniline, N-aryl-aniline, N heterocylclyl-aniline or an amide in step a). To derivatize compound (2) to an N-alkyl-aniline, it may be reacted with several alkylating compounds with different methods, which are easily available in the literature for an expert, such as: alcohols with catalysis of mixture of metals such as copper, palladium, zinc, aluminium, Barium, manganese, chromium, titanium, gold, vanadium, silver, etc. or the correspondent metal oxides with optionally the presence of hydrogen atmosphere; alkylating agents such as aldehydes, cyanides, trimethyl or triethyl orthoesters optionally with the presence of acidic catalysis of trifluoroacetic acid (TFA), H₂SO₄, sulfurochlorhydric acid (HSClO₃), phosphoric acid (H₃PO₄), etc. with a following reduction with borane, borohydride derivatives or catalytic hydrogenation; alkyl sulfonates, alkyl sulfates or alkyl halides (Cl, Br, I) with a suitable base such as sodium carbonate (Na₂CO₃), K₂CO₃, caesium carbonate (Cs₂CO₃), TEA, DIPEA, butyl lithium (BuLi), etc. with optionally with catalysts such as 3-alkyl-1-methyl-*H*-Imidazolium iodide, etc. All these reactions must be performed in a suitable solvent such as: metanol (MeOH), etanol (EtOH), DCM, DCE, EtOAc, THF, 1,4-dioxane, DMF, Et₂O, dimethylsufoxide (DMSO), toluene, xylene, etc. in a standard reactor between 10 and 200ºC. To derivatize compound (2) to an N-aryl-aniline, it may be reacted with several arylating compounds with different methods, which are also easily available in the literature for an expert, such as: aryl halides (F, Cl, Br, I), aryl *p*-toluenesulfonates, aryl sulfamates, diaryliodonium salts, arylboronic acids, tretraarylstiboranyl salts or triarylbismuth (V) salts or tetrarylbismuth (IV) with bases such as lithium, sodium or potassium *t*-butoxide, sodium acetate, Cs₂CO₃, NaH, KOH, etc. in presence of catalytic palladium, nickel, copper or gold complexes. All these reactions can be performed in a suitable solvent such as: metanol (MeOH), etanol (EtOH), DCM, DCE, EtOAc, THF, 1,4-dioxane, DMF, Et₂O, dimethylsufoxide (DMSO), toluene, xylene, etc. in a standard reactor between 10 and 200ºC. Methods that may be used to derivatise compound (2) to an N-heterocyclyl-aniline depend on the aromaticity of the heterocycle. For aliphatic heterocyclyls may be used the same methods as exposed for alkyls, and for heteroaryls, the same exposed for aryls.

Alternatively, a compound of formula (4) with R₇ ≠ H can be obtained when (2) and (3) react in a 1:2 molecular ratio, i.e. di-addition, whereas with R₇=H, it is obtained by reaction of (2) and (3) in a 1:1 molecular ratio, i.e. monoaddition. Thus, depending on the reaction conditions, which may be easily determined by the skilled person, an amide or an imide of formula (4) is obtained. If R₇ = CO-(C₁-C₅) alkyl or - R₇ = CO-(C₁-C₅) alkyl different to CO-R₈, in the step a) may be used the same methods and conditions as in step b). Alternatively, compound (4) can be obtained from (2) by a reaction with one equivalent of a dicarboxylic compound or a derivative (3) thereof.

According to step c), compounds of general formula (5) may be prepared by reducing the nitrocompound of general formula (4) with metals or metal salts such as zinc, iron, tin, tin (II) chloride, etc., in a solvent such as DCM, EtOAc, EtOH, MeOH, isopropyl alcohol (IPA), THF, DMF, AcOH, etc., optionally in the presence of acid, such as aqueous HCl, AcOH, H₂SO₄, etc. in a standard reactor or flask between -30ºC and 100ºC. Alternatively, compounds of formula (5) can be obtained by reaction of a compound of general formula (4) in a hydrogen atmosphere with a pressure between 1 and 5 bar in presence of catalysts such as palladium, rhodium, platinum, copper, vanadium, iron, etc., optionally mixed, in solvents such as MeOH, EtOH, IPA, water (H₂O), THF, DMF, 1,4-dioxane etc. in a standard hydrogenation reactor between 0ºC and 50ºC. Alternatively, compounds of formula (5) can be obtained by reaction of a compound of general formula (4) with sodium sulfide, sodium hydrogensulfide or other sulfide salts, optionally hydrates, in solvents such as MeOH, EtOH, IPA, H₂O, THF, DMF, 1,4-dioxane etc. in a standard reactor between 0ºC and 150ºC.

Then, azocompounds of general formula (6) may be prepared by reacting a compound of formula (5) with a nitrosoderivative of pyridine (R₁-NO, (7a)), in a solvent such as DCM, DCE, EtOAc, THF, DMF, 1,4-dioxane, toluene, etc. optionally in the presence of a base such as sodium hydroxide (NaOH), potassium hydroxide (KOH), alumina, etc. or acidic catalysis such as formic acid, TFA, AcOH, HCl, H₂SO₄, etc. in a standard reactor or flask between 0ºC and 100ºC. An alternative method is reacting a compound of general formula (5) with sodium nitrite and an acid such as HCl, H₂SO₄, etc.; in mixtures of water and MeOH, EtOH, IPA, THF, DMF, etc. in a standard reactor or flask between - 30ºC and 30º. The resulting diazonium salt which is treated with a pyridine (R₁-H, (7b)) and a base such as NaOH, KOH, K₂CO₃, sodium hydrogencarbonate (NaHCO₃), tetramethyl ammonium hydroxide, tetramethyl ammonium carbonate, sulfamic acid, etc. between -20ºC and 75ºC to give the azocompounds of general formula (6).

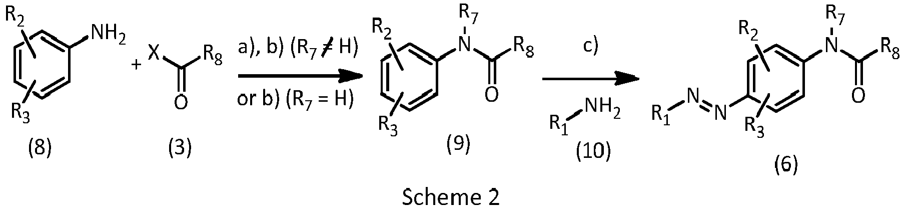

Alternatively, compounds of formula (6) as defined above in Scheme 1 may be prepared as shown in Scheme 2, where the steps of optional derivatisation of an aniline (step a)), formation of amide or imide (step b)) follow the same synthetic methods as shown in Scheme 1 and explained above. For the azobond formation (step c)), compounds of general formula (6) may be prepared reacting a compound of general formula (10) with sodium nitrite and an acid such as HCl, H₂SO₄, etc.; in mixtures of water and MeOH, EtOH, IPA, THF, DMF, etc. in a standard reactor or flask between -30ºC and 30º. The resulting diazonium salt which is treated with a compound of general formula (9) and a base such as NaOH, KOH, K₂CO₃, KHCO₃, tetramethyl ammonium hydroxide, tetramethyl ammonium carbonate, sulfamic acid, etc. between -20ºC and 75ºC to give the azocompounds of general formula (6).

Alternatively, compounds of formula (6) as defined above in Scheme 1 may be prepared as shown in Scheme 3, where the steps of reduction (step b)), optional derivatisation of the aniline (step c)), azo-bond formation (steps c') and f)) and amide or imide formation (steps d) and f')) follow the synthetic methods for the same type of reactions as shown in Scheme 1 and explained above.

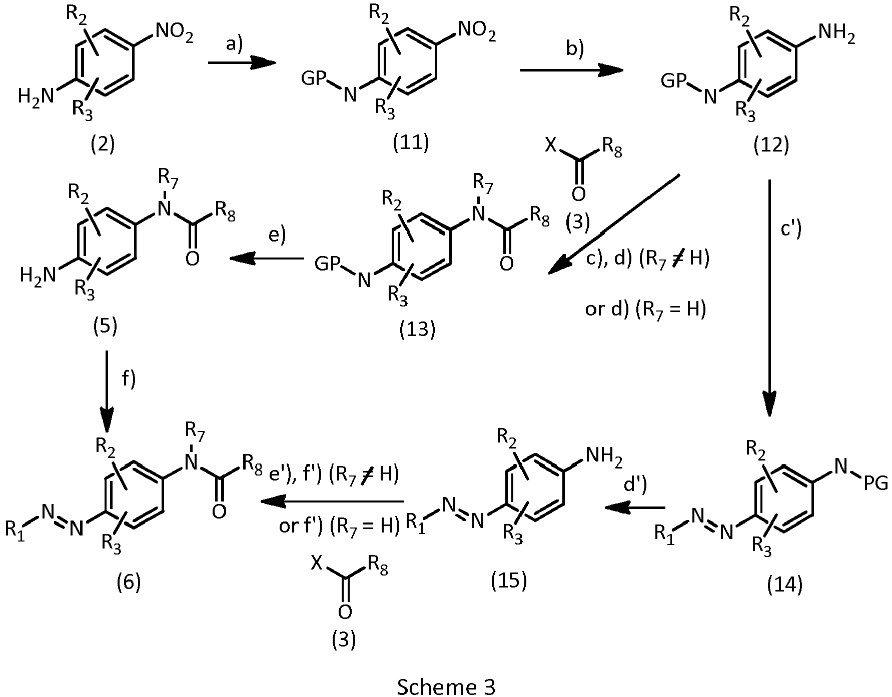

At the protection step, (step a)), compounds of formula (11) may be formed by protection of the aniline group of compounds of general formula (2) with a standard amino protecting group (PG) known to those skilled in the art, such as tert-butyloxycarbonyl (Boc), carbobenzyloxy (Cbz), p-methoxybenzyl carbonyl (Moz), or other carbamates; benzyl (Bn), benzoyl (Bz), phtalimide, p-methoxybenzyl (PMB), p-methoxyphenyl (PMP), 9-fluorenylmethyloxycarbonyl (Fmoc), tosyl (Ts), etc. [see Greene, T.W., Wuts, P.G.M. Protective Groups in Organic Synthesis (Third Edition), J. Wiley & Sons, 1999]. These compounds (11) may be prepared from compounds of general formula (2) reacting with the corresponding protecting agent in solvents such as DCM, DCE, EtOAc, THF, DMF, 1,4-dioxane, toluene, water, AcOH, etc., in a standard reactor or flask between 0ºC and 150ºC.

At deprotection steps (steps e) and d')), compounds of general formula (5) and (15) may be prepared by deprotection of the aniline group of compounds of general formula (13) and (14), respectively. The method used depends on the present protecting group and it is known to those skilled in the art [see Greene, T.W., Wuts, P.G.M. Protective Groups in Organic Synthesis (Third Edition), J. Wiley & Sons, 1999]. The preparation consists on reacting compounds of general formula (13) or (14) with suitable reagents such as HCl, hydrobromic acid (HBr), H₂SO₄, TFA, piperidine, NaH, ammonia (NH₃), methylamine (MeNH₂), ammonium cerium (IV) nitrate, sodium in liquid ammonia, hydrogen with a hydrogenation catalyst (palladium, rhodium, platinum, copper, vanadium, iron...), etc. in a suitable solvent such as DCM, DCE, MeOH, EtOAc, THF, DMF, 1,4-dioxane, toluene, water, AcOH, etc., in an adequate reactor or flask between -78ºC and 200ºC.

Compounds of formula (19), i.e. compounds of formula (1) wherein R₄ is -NHCO-NHR₁₀, can be prepared following the synthetic pathway depicted in Scheme 4, comprising the optional derivatisation of an aniline (2) (step a)), formation of carbamide (17) by reaction of an aniline of formula (2) (derivatised or not) and isocyanate, carbamate or other aminocarbonyl compounds or their precursors (16) (step b)), followed by reduction of the nitro group of the obtained compound (17) to give an amino derivative of formula (18) (step c)) and final azo-bond formation to give the compounds of formula (19) (step d)).

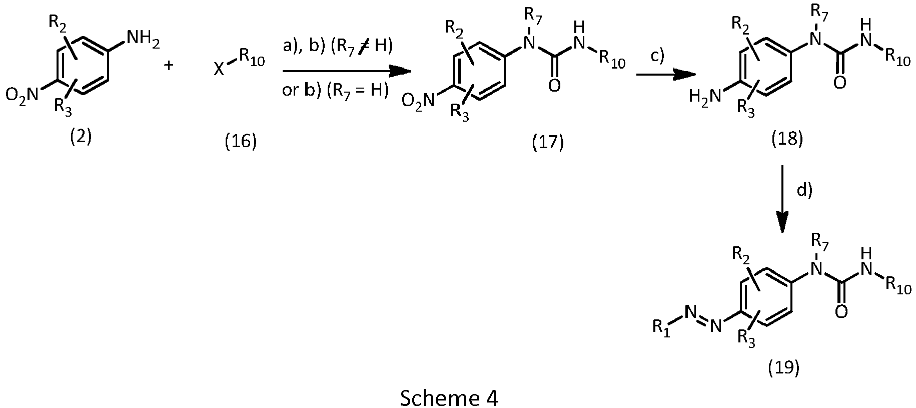

Thus, in step b) carbamides of formula (17) may be prepared by reacting an aniline of formula (2) (derivatized or not) with an organic isocyanate or organic carbamate (16) suitable for the synthesis of carbamides, obtained from a commercial source or prepared *in situ.* The reaction may be carried out in one pot in a solvent such as DCM, DCE, EtOAc, THF, 1,4-dioxane, DMF, AcOH, Et₂O, acetonitrile (MeCN), toluene, xylene, etc., optionally in the presence of one or more coupling agents such as EDC, DIC, DCC, N,N'-di-tert-butylcarbodiimide, bis(trimethylsilyl)carbodiimide, etc. in a presence of a base such as TEA, DIPEA, Hünig's base, NaH, K₂CO₃, alumina, etc. in a standard reactor or flask between 0ºC and 150ºC. Optionally catalysts such as DMAP may be used.

Alternatively, other compounds (16) suitable for the synthesis of carbamides can be used, such as: organic carbonates, carbonyl diimidazole, phosgene, diphosgene, triphosgene, etc. in similar conditions as explained above. Steps a), c) and d) are carried out following the synthetic methods for the same type of reactions as shown in Scheme 1 and explained above.

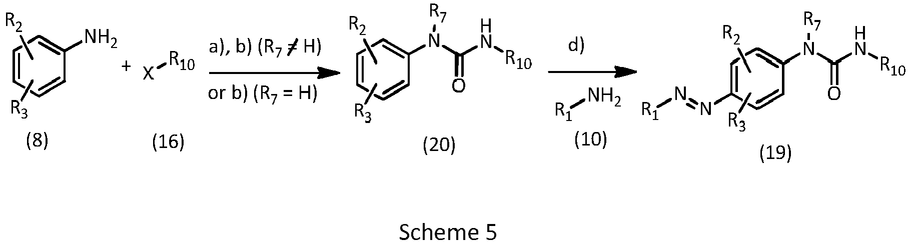

Alternatively, compounds of formula (19) may be prepared as shown in Scheme 5, where the steps of optional derivatisation of an aniline (8) (step a)), and formation of a carbamide (20) (step b)) follow exactly the same synthetic methods as shown in Scheme 4 and explained above; and the step of the azobond formation (step c)) follow exactly the same synthetic methods as shown in Scheme 2.

Alternatively, compounds of formula (19) may be prepared as shown in Scheme 6 following the same protocol as described in Scheme 3, except the steps d) and f') to obtain carbamides (21) and (19), respectively, which are carried out by reaction with an isocyanate, carbamate or other amino carbonyl or their precursors (16) following the synthetic methods described above to obtain carbamide (17) in Scheme 4.

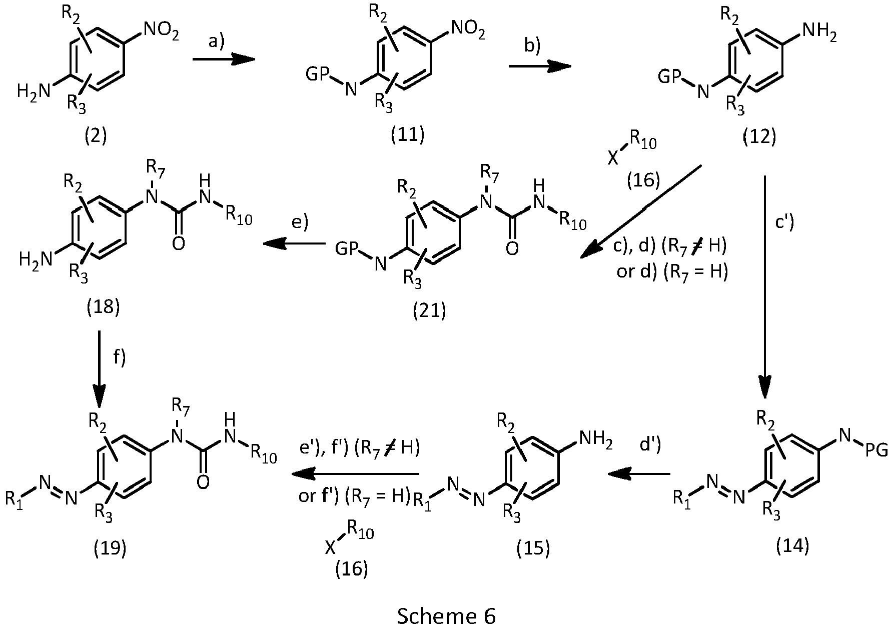

Compounds of formula (25), i.e. compounds of formula (1) wherein R₄ is -NHSO₂-R₉ can be prepared following the synthetic pathway depicted in Scheme 7 comprising the optional derivatisation of an aniline (2) (step a)), formation of a sulfonamide (23) from an aniline of formula (2) (derivatised or not) and a compound of formula (22) (step b)), reduction of nitro group in the compound of formula (23) to give an amino derivative (24) (step c)), and azo-bond formation to give the sulfonamides of formula (25) (step d)).

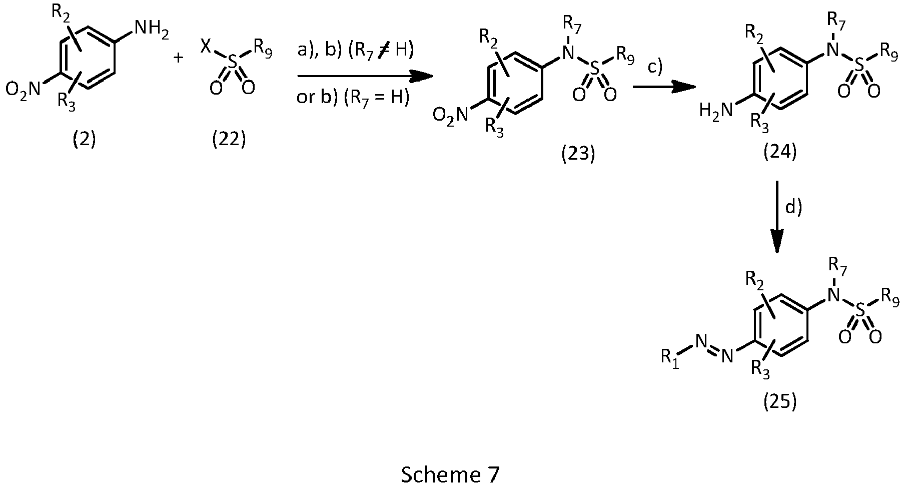

Thus, in step b) compounds of general formula (23) (derivatized or not), may be prepared by reacting an aniline of formula (2) with a compound (22) selected from organic sulfonyl chloride, sulfonic acid, etc. commercially available o prepared in situ in a solvent such as DCM, DCE, chloroform, water, EtOAc, THF, DMF, 1,4-dioxane, toluene, etc.), optionally in a presence of a base such as TEA, DIPEA, ,Na₂CO₃, K₂CO₃, NaOH, pyridine, etc. and optionally a presence of an acid such as HCl, H₂SO₄, formic acid, etc. in a standard reactor or flask between 0ºC and 150ºC. Optionally catalysts such as DMAP may be used. Steps a), c) and d) are carried out following the synthetic methods for the same type of reactions as shown in Scheme 1 and explained above.

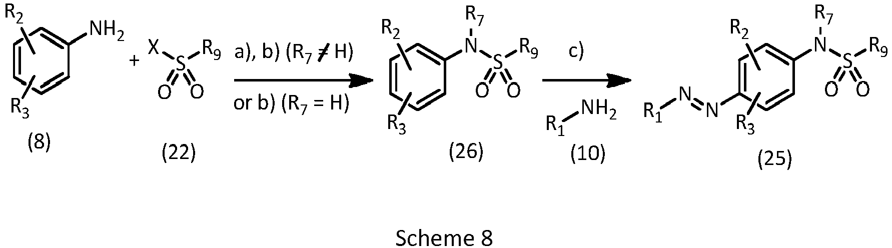

Alternatively, compounds of formula (25) may be prepared as shown in Scheme 8, where the steps of optional derivatisation of an aniline (8) (step a)), and formation of sulfonamide (step b)) follow exactly the same synthetic methods as shown in Scheme 7 and explained above; and the step of the azobond formation (step c)) follows the synthetic methods for the same type of reactions as shown in Scheme 2 and explained a bove.

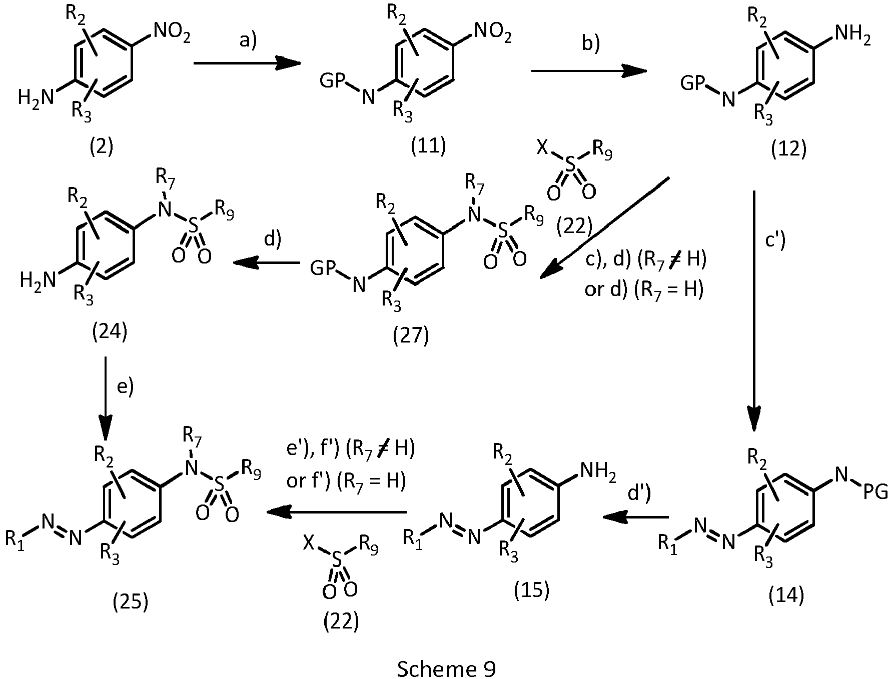

Alternatively, compounds of formula (25) may be prepared as shown in Scheme 9 following the same protocol as described in Scheme 3, except steps d) and f') to obtain sulfonamides (27) and (25), respectively, which are carried out by reaction with a sulfonyl compound (22), as defined above in Scheme 7.

Compounds of formula (30), i.e. compounds of formula (1) wherein R₄ is -CONH-, may be prepared following the synthetic procedure depicted in Scheme 10, comprising azobond formation (step a)), followed by a amide or imide (step b)) formation.

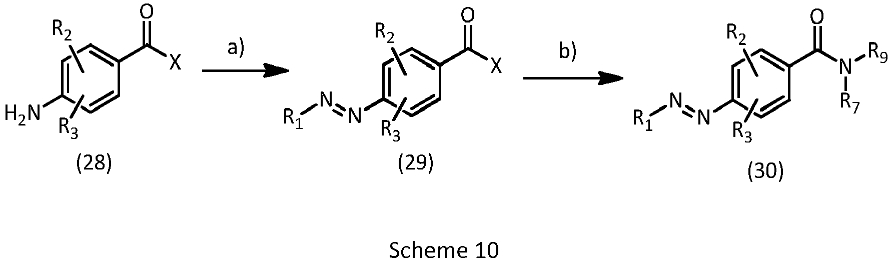

According to Scheme 10, compounds of general formula (29), where X can be OH, SH, halide or any other activating group such as 1*H*-benzo[d][1,2,3]triazol-1-oxy, N-succinimidyloxy, 7-azabenzotriazol-1-oxy, 4-nitrophenyloxy, 1*H*-imidazol-1-oxy, 1,3-dioxo-3a,4,7,7a-tetrahydro-1*H*-4,7-methanoisoindol-2(3*H*)yl-oxy, etc., are obtained from a compound of formula (28) with a compound of general formula (7) following the methodology described above for Scheme 1.

Thus, compounds of general formula (30) as defined above may be prepared by reacting a compound of general formula (29) as defined above, with an amine derivative (R₉-NH₂, or R₉-NH-R₇), in a solvent such as DCM, DCE, MeOH, EtOAc, THF, DMF, 1,4-dioxane, toluene, water, AcOH, etc., optionally in the presence of more coupling agents such as described above, or with previous activation using activating agents like thionyl chloride (SOCl₂), oxalyl chloride, acetyl chloride, isobuthyl chloroformate, etc., in a presence of a base such as TEA, DIPEA, Hünig's base, NaH, K₂CO₃, alumina, etc. in a standard reactor or flask between 0ºC and 150ºC. Optionally catalysts such as DMAP may be used.

Alternatively, compounds of formula (30), can be synthesized following an inverse order of the reactions, forming the amide (step b)) prior to the azobond formation (step a)).

In the following synthetic schemes, the compounds according to the present invention and their synthetic intermediates and precursors are depicted with the corresponding R₄ group in meta position to the corresponding R₁-N=N moiety, but they do also encompass the compounds of the present invention and their synthetic intermediates and precursors wherein the corresponding R₄ is in the para position with respect to the corresponding R₁-N=N moiety, starting from the corresponding p-diaminobenzenes.

Compounds of general formula (33), i.e. compounds of formula (1) wherein R₄ is - NR₇CO-R₉ and is located in the meta position in respect to the R₁-N=N- moiety, can be prepared following the synthetic pathway depicted in Scheme 11.

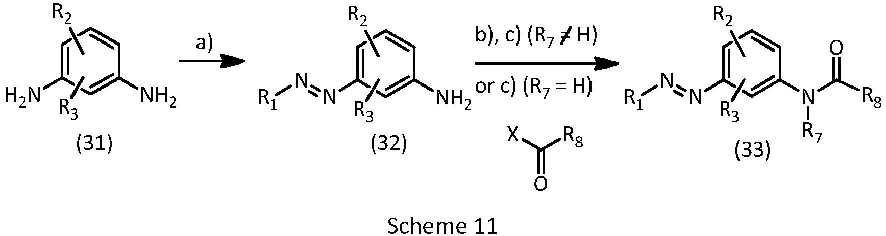

The compounds with general formula (32) and (33) may be prepared from compounds of formula (31) following the experimental methods of azo-bond formation (step a)), the optional aniline derivatisation (step b)) and amide or imide formation (step c)), which have been explained above, e.g. regarding the steps d), a) and b) respectively in Scheme 1.

Compounds of general formula (34), i.e. compounds of formula (1) wherein R₄ is-NR₇CO-NHR₁₀, can be prepared following the synthetic pathway depicted in Scheme 12

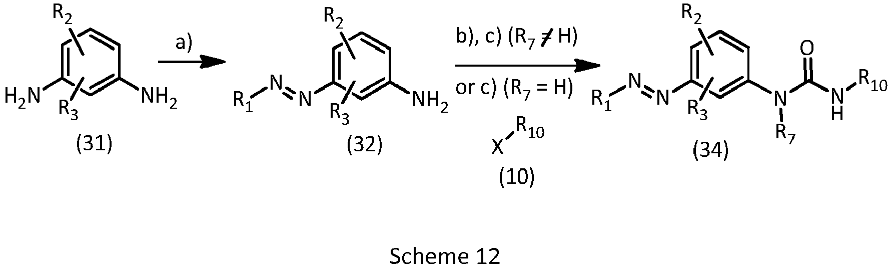

The compounds with general formula (32) and (34) may be prepared from compounds of formula (31) following the experimental methods of azo-bond formation (step a)), the optional aniline derivatisation (step b)) and carbamide formation (step c)), which have been explained above, e.g. regarding the steps d), a) and b) respectively in Scheme 4.

Compounds of general formula (35), i.e. compounds of formula (1) wherein R₄ is - NHSO₂-R₉, can be prepared following the synthetic pathway depicted in Scheme 13.

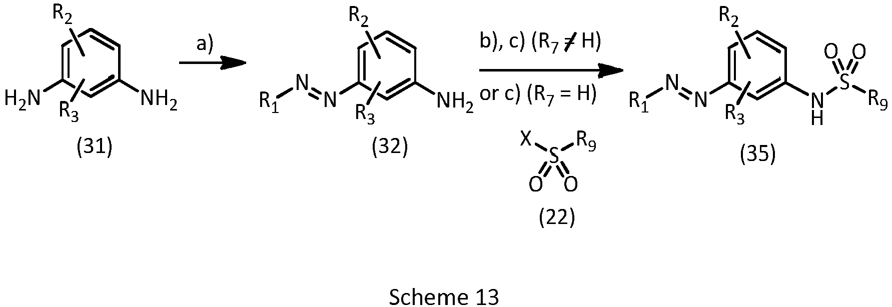

The compounds with general formula (32) and (35) may be prepared from compounds of formula (31) following the experimental methods of azo-bond formation (step a)), the optional aniline derivatisation (step b)) and sulfonamide formation (step c)) which have been explained above, e.g. regarding the steps d), a) and b) respectively in Scheme 7.

Compounds of general formula (40), i.e. compounds of formula (1) wherein R₄ is -S-R₉ or -O-R₉ may be prepared following the synthetic scheme depicted in Scheme 14 comprising formation of ether or thioether of formula (38), step a), from the compound of formula (36), wherein Y is O or S, followed by reduction of the nitro group of the compound of formula (38) to give the aniline of formula (39), step b), and azo-bond formation to give the compounds of formula (40), step c). The steps of reduction of the compound of formula (38), step b), and azo-bond formation from the compound of formula (39) to give compounds (40), step c), following the synthetic methods for the same type of reactions as shown in Scheme 1 and explained above.

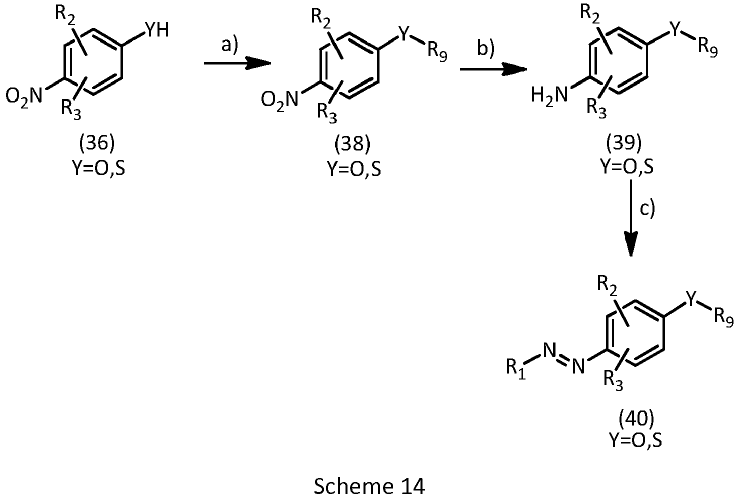

Thus, compounds of general formula (38) may be obtained by Williamson ether synthesis, by Mitsunobu reaction or Ullmann coupling. In the first case, compound of formula (36) may be reacted with a compound (R₉-Z, 37a), wherein Z is a suitable leaving group such as halogen, including Cl, Br, I, or sulfonate ester, including mesylate, tosylate, nosylate etc. This reaction is carried out in the presence of a base such as NaOH, KOH, K₂CO₃, Cs₂CO₃, etc. with optionally the presence of quaternary ammonium salts ore iodine salts in a suitable solvent such as DMF, EtOAc, THF, DMSO, MeOH, MeCN, etc., in a standard reactor or flask between 0ºC and 150ºC. Alternatively, compound of formula (36) may be reacted with diaryliodinium salts (37b) under the same conditions.

The Mitsunobu reaction comprise a compound of formula (36) that is reacted with a compound with a hydroxyl group (R₉-OH, 37c) in presence of reagents such as triphenylphosphine, trimethylphosphine or polymer-supported triphenylphosphine and diethyl azodicarboxilate (DEAD), diisopropyl azodicarboxilate (DIAD), tetramethyl azodiamide (TMAD), 1,1'-(azodicarbonyl)dipiperidine (ADDP), azopyridine, etc. in one pot in a suitable solvent such as THF, DCM, DCE, EtOAc, MeCN, etc., in a standard reactor or flask between 0ºC and 150ºC. Optionally microwave irradiation may be used.

The Ullmann coupling involves the same conditions of Williamson ether synthesis, but using compound (R₉-Z, 37a), wherein Z is a suitable leaving group and R₉ an aromatic ring and employing a copper compound such as copper powder, CuO, CuI, Cu₂O. Cu(OAc)₂, etc. or copper complexes heating to temperatures between 50-250ºC.

Alternatively, compounds of formula (40) may be prepared according to the synthetic pathway depicted in Scheme 15, where steps a) and b) correspond to steps c) and a) in scheme 14 respectively.

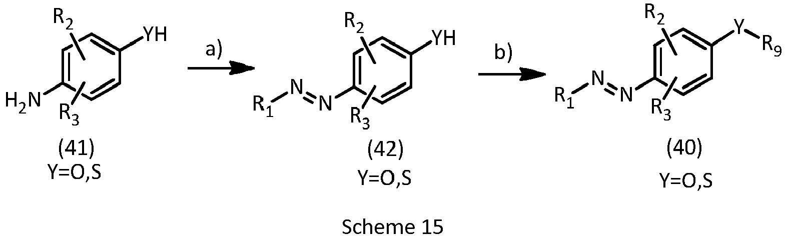

### Pharmacological activity

The mGluRs and specifically mGlu5 and mGlu4 are considered a potential drug target for treatment of psychiatric and neurological disorders. The compounds of formula (1) according to the present invention may be used for treating or preventing a condition in a mammal, including a human, the treatment or prevention of which is affected or facilitated by the neuromodulatory effect of mGlu5 allosteric ligands controlled by light.

Therefore, in a second aspect, the present invention provides a compound of formula (1) as defined above, or a pharmaceutically acceptable salt, stereoisomer or solvate thereof, for use as a medicament.

The invention also relates to the use of a compound of formula (1) as defined above, or a pharmaceutically acceptable salt, stereoisomer or solvate thereof, for the manufacture of a medicament.

In a third aspect, the present invention provides a compound of formula (1) as defined above, or a pharmaceutically acceptable salt, stereoisomer or solvate thereof, for use in the treatment and/or prevention of a disease or condition selected from the group consisting of psychosis, epilepsy, schizophrenia, Alzheimer's disease, cognitive disorders, memory deficits, Parkinson's disease, hypoxia, ischemia, dementia caused by AIDS, migraine, depression, mood disorders, anxiety disorders, fragile X syndrome, pain syndromes (such as neuropathic pain and chronic pain), addictive disorders (such as drug or alcohol abuse or addiction and substance tolerance or dependence, and drug withdrawal), bulimia nervosa, anorexia nervosa, gambling dependence, Ophelia syndrome, smoking, sex dependence and obsessive compulsive disorders, and stomach diseases (such as gastroesophageal reflux); preferably pain syndromes (such as neuropathic pain and chronic pain), Parkinson disease, drug withdrawal and fragile X syndrome.

The present invention relates to the use of a compound of formula (1) as defined above, or a pharmaceutically acceptable salt, stereoisomer or solvate thereof, for manufacturing a medicament for the treatment and/or prevention of a disease or condition selected from the group consisting of psychosis, epilepsy, schizophrenia, Alzheimer's disease, cognitive disorders, memory deficits, Parkinson's disease, hypoxia, ischemia, dementia caused by AIDS, migraine, depression, mood disorders, anxiety disorders, fragile X syndrome, pain syndromes (such as neuropathic pain and chronic pain), addictive disorders (such as drug or alcohol abuse or addiction and substance tolerance or dependence and drug withdrawal), bulimia nervosa, anorexia nervosa, gambling dependence, smoking, sex dependence and obsessive compulsive disorders, stomach diseases (such as gastroesophageal reflux), and Ophelia syndrome preferably pain syndromes (such as neuropathic pain and chronic pain), Parkinson disease, drug withdrawal and fragile X syndrome.

The invention also relates to a method of treatment and/or prevention of a subject in need thereof of a disease or condition selected from the group consisting of psychosis, epilepsy, schizophrenia, Alzheimer's disease, cognitive disorders, memory deficits, Parkinson's disease, hypoxia, ischemia, dementia caused by AIDS, migraine, depression, mood disorders, anxiety disorders, fragile X syndrome, pain syndromes (such as neuropathic pain and chronic pain), addictive disorders (such as drug or alcohol abuse or addiction and substance tolerance or dependence, and drug withdrawal), bulimia nervosa, anorexia nervosa, gambling dependence, smoking, sex dependence and obsessive compulsive disorders, stomach diseases (such as gastroesophageal reflux), and Ophelia syndrome, preferably pain syndromes (such as neuropathic pain and chronic pain), Parkinson disease, drug withdrawal and fragile X syndrome ,which comprises the administration of a therapeutically effective amount of a compound of formula (1) as defined above.

### Pharmaceutical compositions/formulations and administration

In a fourth aspect the invention is directed to a pharmaceutical composition comprising a compound of formula (1) as defined above, or a pharmaceutically acceptable salt, stereoisomer or solvate thereof, and a pharmaceutically acceptable excipient.

The term "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similar. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005.

Compounds formula (1) according to the present invention may be administered by the oral, sublingual, parenteral, subcutaneous, intramuscular, intravenous, transdermal, intranasal, intraocular, and/or rectal routes. The compounds may be administered alone or in combination with one or more other compounds of the invention or one or more other drugs. In general, the compounds should be administered as a formulation in association with one or more pharmaceutically acceptable excipients. Any administration method commonly used for drugs, such as tablets, coated tablets, capsules, solution (including nanoemulsion), syrup, powder, suppository, cream, and ointment, may be used. The pharmaceutical composition can be formulated employing conventional liquid or solid vehicles or diluents and pharmaceutical additives, according to the desired mode of administration. The mentioned formulations will be prepared using standard methods, such as those described or referred to in the Spanish and U.S. Pharmacopoeias and similar reference texts.

The following examples represent specific embodiments of the present invention. They do not intend to limit in any way the scope of the invention defined in the present description.

### Examples

### Materials and methods

All the Chemicals and solvents are from commercial suppliers and used without purification, except the anhydrous solvents such as DMF which were treated previously through a system of solvent purification (*PureSolv*)*,* degasified with inert gases and dried over alumina or molecular sieves; dry TEA was distilled over calcium hydride. Reactions were monitored by thin layer chromatography (60 F, 0.2 mm, *Macherey-Nagel)* by visualisation under 254 and/or 365 nm lamp. Flash column chromatography *Panreac Silica Gel 60, 40-63 microns RE* or *Biotage SNAP KP Sil 50 microns,* automatized with Biotage Isolera One with UV-Vis detection. Nuclear magnetic resonance spectrometry (NMR) *Variant-Mercury 400* MHz. Chemical shifts δ are reported in parts per million (ppm) against the reference compound tetramethylsilane using the signal of the residual non-deuterated solvent (Chloroform δ = 7.26 ppm (¹H), δ = 70 ppm (¹³C)). High-performance Liquid Chromatography (HPLC) *Alliance Waters 2695* separation module coupled to *Waters 2996* photodiode detector (DAD), 5 µL of sample 2.5 mM in DMSO was injected, using a *Kinetex* C18 2.6 µm 4.6 x 50 mm *(Phenomenex)* column. The mobile phase used was a mixture of A = NaH₂PO₄/ Na₂HPO₄ aqueous buffer pH=7 and B = methanol, with method described as follows: flow 1 mL/min 5% B-70% B 1 min, 70% B-80% B 1 min, 80% B-100% B 6 min, runtime 12 min. Mass spectroscopy (MS) analysed by FIA (flux injected analysis) with *Waters Aquity* coupled to *LCT Premier Orthogonal Accelerated Time of Flight Mass Spectrometer,* acquiring data by electrospray ionisation (ESI) in positive mode. Spectra have been scanned between 50 and 1500 Da with values every 0.2 seconds and peaks are given m/z (% of basis peak).

### A. Preparation of intermediates

### Example 1. Preparation of compounds of formula (4):

### Method A

To a dispersion of the corresponding aniline of formula 2 (1 eq) and the corresponding acylating agent of formula **3** (1.1-2.2 eq.) in DCE, DIPEA was added. The resulting mixture was heated at 100ºC in a corresponding reactor under stirring between 1-4 h and was diluted in DCM (12 mL). The resulting solution was washed with saturated NaHCO₃ (10 mL), Na₂CO₃ (10 mL), water (10 mL) and brine (10 mL), dried over anhydrous Na₂SO₄, filtered and evaporated to dryness. The obtained solid was used without further purification or was purified through silica flash column (hexane/EtOAc).

According to this methodology, using the suitable compounds of formulae **2** and **3,** compounds **(4a)-(4c)** were prepared.

**Table 1**

| Prod. | Comp. (2) | Comp. (3) | mmol DIPEA | solvent | Time | Purif. | Yield |
|---|---|---|---|---|---|---|---|
| **4a** | 2-methoxy-4-nitroaniline 0.6 mmol | 4-benzoylbenzoyl chloride 0.7 mmol | 1.3 | DCE 3.0 mL | 1 h | no | 100% |
| **4b** | 2-methoxy-4-nitroaniline 0.6 mmol | 2,4-dichlorobenzoyl chloride 1.7 mmol | 2.6 | DCE 3.0 mL | 4 h | yes | 67% |
| **4c** | 2-methoxy-4-nitroaniline 3 mmol | 2-chlorobenzoyl chloride 3.2 mmol | 6.4 | DCE 4.5 mL | 1h | no | 90% |

### 4-Benzoyl-N-(2-methoxy-4-nitrophenyl)benzamide (4a):

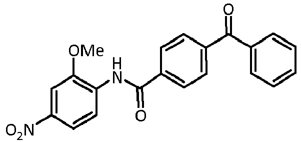

¹H NMR (400 MHz, CDCl₃) δ 8.80 (s, 1H), 8.76 (d, *J* = 9.0 Hz, 1H), 8.04 - 7.98 (m, 3H), 7.96 - 7.91 (m, 2H), 7.85 - 7.79 (m, 3H), 7.68 - 7.60 (m, 1H), 7.52 (t, *J* = 7.6 Hz, 2H), 4.08 (s, 3H).

### 2,4-Dichloro-N-(2-methoxy-4-nitrophenyl)benzamide (4b):

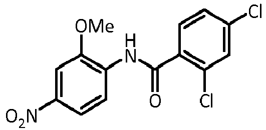

¹H NMR (400 MHz, CDCl₃) δ 8.90 (s, 1H), 8.73 (d, *J* = 9.0 Hz, 1H), 7.98 (dd, *J* = 9.0, 2.4 Hz, 1H), 7.80 (dd, *J* = 5.4, 2.9 Hz, 2H), 7.52 (d, *J* = 2.0 Hz, 1H), 7.41 (dd, *J* = 8.4, 2.0 Hz, 1H), 4.02 (s, 3H).

### 2-Chloro-N-(2-methoxy-4-nitrophenyl)benzamide (4c):

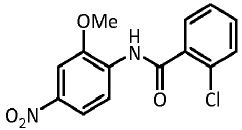

¹H NMR (400 MHz, CDCl₃) δ 8.88 (s, 1H), 8.75 (d, *J* = 9.0 Hz, 1H), 7.98 (dd, *J* = 9.0, 2.4 Hz, 1H), 7.84 - 7.80 (m, 1H), 7.79 (d, *J* = 2.4 Hz, 1H), 7.52 - 7.38 (m, 3H), 4.01 (s, 3H).

### Method B

To a dispersion of the corresponding aniline of formula **2,** the corresponding acylating agent of formula 3 and HATU in a suitable solvent, DIPEA was added and the resulting mixture was stirred a temperature between 18-64 h at 40-80ºC. Afterwards, the dispersion was diluted in EtOAc (12 mL) and washed with NaOH or Na₂CO₃ 1N (10 mL), saturated NaHCO₃ (10 mL), water (10 mL) and brine (10 mL), dried over anhydrous MgSO₄, filtered and evaporated to dryness. The obtained solid was purified through silica flash column (hexane/EtOAc).

According to this methodology, using the suitable compounds of formulae **2** and **3,** the following compounds **(4d)-(4h)** were prepared, as shown in Table 2.

**Table 2**

| Prod | Comp. 2 | Comp.3 | mmol HATU | mmol DIPEA | Solvent | Temp. | Time | Yield |
|---|---|---|---|---|---|---|---|---|
| **4d** | 2-methoxy-4-nitroaniline 0.5 mmol | Picolinic acid 1 mmol | 1 | 1 | EtOAc 6.0 mL | 45ºC | 19 h | 87% |
| **4e** | 2-methoxy-4-nitroaniline 0.3 mmol | Nicotinic acid 0.5 mmol | 0.7 | 1 | DMF 1.0 mL | 80ºC | 64 h | 74% |
| **4f** | 2-methoxy-4-nitroaniline 0.3 mmol | Isonicotinic acid 0.5 mmol | 0.7 | 1 | DMF 1.0 mL | 80ºC | 64 h | 72% |
| **4g** | 2-cyano-4-nitroaniline 0.4 mmol | Picolinic acid 0.5 mmol | 1.0 | 1 | DMF 1.0 mL | 40ºC | 24 h | 85% |
| **4h** | 4-nitroaniline 0.5 mmol | Picolinic acid 0.7 mmol | 1.5 | 1.5 | DMF 1.5 mL | 40ºC | 18 h | 94% |

### N-(2-methoxy-4-nitrophenyl)picolinamide (4d):

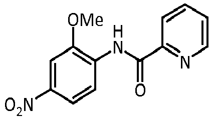

¹H NMR (400 MHz, CDCl₃) δ 10.82 (s, 1H), 8.79 (d, J = 9.0 Hz, 1H), 8.28 (dd, J = 4.9, 3.9 Hz, 1H), 8.06 - 7.86 (m, 2H), 7.79 (d, J = 2.4 Hz, 1H), 7.52 (ddd, J = 7.6, 4.8, 1.2 Hz, 1H), 4.08 (s, 3H).

### N-(2-methoxy-4-nitrophenyl)nicotinamide (4e):

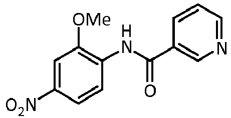

¹H NMR (400 MHz, CDCl₃) δ 9.14 (d, J = 1.9 Hz, 1H), 8.83 (dd, J = 4.8, 1.5 Hz, 1H), 8.73 (m, 2H), 8.25 (ddd, J = 8.0, 2.3, 1.7 Hz, 1H), 8.00 (dd, J = 9.0, 2.4 Hz, 1H), 7.82 (d, J = 2.5 Hz, 1H), 7.50 (ddd, J = 8.0, 4.9, 0.8 Hz, 1H), 4.07 (s, 3H).

### N-(2-methoxy-4-nitrophenyl)isonicotinamide (4f):

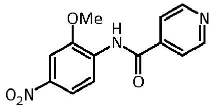

¹H NMR (400 MHz, CDCl₃) δ 8.85 (dd, J = 4.4, 1.6 Hz, 2H), 8.75 (s, 1H), 8.72 (d, J = 9.0 Hz, 1H), 7.99 (dd, J = 9.0, 2.4 Hz, 1H), 7.82 (d, J = 2.4 Hz, 1H), 7.73 (dd, J = 4.4, 1.7 Hz,2H), 4.07 (s, 3H).

### N-(2-cyano-4-nitrophenyl)picolinamide (4g):

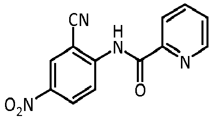

¹H NMR (400 MHz, CDCl₃) δ 11.25 (s, 1H), 9.02 (d, J = 9.3 Hz, 1H), 8.74 (ddd, J = 4.8, 1.7, 0.9 Hz, 1H), 8.55 (d, J = 2.6 Hz, 1H), 8.50 (ddd, J = 9.3, 2.6, 0.6 Hz, 1H), 8.31 (dt, J = 7.8, 1.1 Hz, 1H), 7.98 (td, J = 7.7, 1.6 Hz, 1H), 7.60 (ddd, J = 7.6, 4.7, 1.2 Hz, 1H).

### N-(4-nitrophenyl)picolinamide (4h):

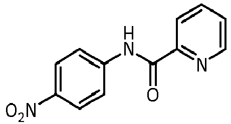

¹H NMR (400 MHz, CDCl₃) δ 8.72 - 8.53 (m, 1H), 8.33 - 8.20 (m, 3H), 8.03 - 7.85 (m, 3H), 7.52 (ddd, J = 7.6, 4.8, 1.2 Hz, 1H).

### Example 2. Preparation of compounds of formula 5

### Method A

A solution of the corresponding nitrocompound of formula 4 and SnCl₂ in EtOH and EtOAc was stirred 5h at reflux temperature and optionally 16 hours at room temperature and it was concentrated. The residue was redissolved in EtOAc (15 mL) and washed with NaOH 2N (10 mL), saturated NaHCO₃ (10 mL), water (10 mL) and brine (10 mL), dried over anhidrous MgSO₄, filtered and concentrated to give a residue which was purified through silica flash column (hexane/EtOAc).

According to this methodology, using the suitable compounds 5, the following compounds (5a) and (5b) were prepared, as shown in Table 3.

**Table 3**

| Prod. | Comp. 4 | mmol SnCl₂ | mL EtOH | mL EtOAc | Temp 1 | Time | Temp 2 | Time | Yield |
|---|---|---|---|---|---|---|---|---|---|
| **5a** | (4a) 0.5 mmol | 1.5 | 10 | 10 | reflux | 5 h | r.t. | 16 h | 84% |
| **5b** | (4b) 0.3 mmol | 0.7 | 5 | 3 | reflux | 2 h | - | - | 70% |

### N-(4-amino-2-methoxyphenyl)-4-benzoylbenzamide (5a):

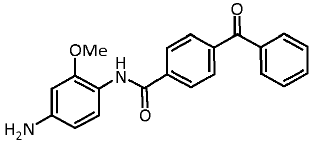

¹H NMR (400 MHz, CDCl₃) δ 8.34 (s, 1H), 8.22 (d, J = 8.4 Hz, 1H), 7.96 (d, J = 8.2 Hz, 2H), 7.87 (d, J = 8.1 Hz, 2H), 7.80 (d, J = 7.8 Hz, 2H), 7.64 - 7.57 (m, 1H), 7.49 (t, J = 7.7 Hz, 2H), 6.32 (dd, J = 12.1, 3.5 Hz, 2H), 3.84 (s, 3H).

### N-(4-amino-2-methoxyphenyl)-2,4-dichlorobenzamide (5b):

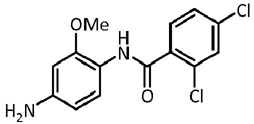

¹H NMR (400 MHz, CDCl₃) δ 8.31 (s, 1H), 8.21 (dd, J = 8.5, 2.2 Hz, 1H), 7.71 (d, J = 8.3 Hz, 1H), 7.45 (d, J = 1.9 Hz, 1H), 7.33 (dd, J = 8.3, 1.9 Hz, 1H), 6.33 (dd, J = 8.5, 2.3 Hz, 1H), 6.29 (s, 1H), 3.82 (s, 3H).

### Method B

To a dispersion of 2-chloro-N-(2-methoxy-4-nitrophenyl)benzamide (4c) (2.6 mmol) and tin (9 mmol) in THF (15 mL) at reflux temperature, it was added dropwise HCl 37% (8 mL) and it is stirred 30 min at reflux temperature. Then, the solution was basified with NaOH 1N, it was added EtOAc (30 mL) and the resulting dispersion was filtered through celite. The layers of the filtrate were separated, and the organic one was washed with water (40 mL) and brine (40 mL), dried over anhydrous MgSO₄, filtered and concentrated to give a residue, which was purified through silica flash column (hexane/EtOAc). 95% yield.

### N-(4-amino-2-methoxyphenyl)-2-chlorobenzamide (5c)

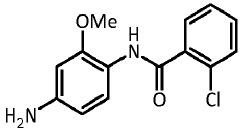

¹H NMR (400 MHz, CDCl₃) δ 8.28 (s, 1H), 8.26 (d, *J* = 8.5 Hz, 1H), 7.75 (dd, *J* = 7.0, 2.3 Hz, 1H), 7.44 (d, *J* = 1.9 Hz, 1H), 7.44 - 7.41 (m, 1H), 7.41 - 7.32 (m, 2H), 6.33 (dd, *J* = 8.5, 2.4 Hz, 1H), 6.29 (d, *J* = 2.4 Hz, 1H), 3.82 (s, 3H).

### Method C

To a dispersion of the corresponding nitrocompound of formula 4 and palladium on carbon in ethanol and 1,4-dioxane was applied a hydrogen atmosphere of 1 bar for 4-5 hours at room temperature. Afterwards, the black dispersion was filtered through Celite, washed with EtOH and 1,4-dioxane (or EtOAc) (2x5 mL each) and the filtrate was evaporated to dryness. The obtained solid was used without further purification or was purified through silica flash column (hexane/EtOAc).

According to this methodology, using the suitable compounds 5, the following compounds (5d)-(5h) were prepared as shown in Table 4.

**Table 4.**

| Prod. | Comp. 4 | mmol Pd/C | mL EtOH | mL 1,4-dioxane | Time | Purif. | Yield |
|---|---|---|---|---|---|---|---|
| **5d** | (4d) 0.4 mmol | 0.2 | 2 | 0 | 4 h | yes | 87% |
| **5e** | (4e) 0.13 mmol | 0.06 | 2 | 0 | 5 h | no | 84% |
| **5f** | (4f) 0.13 mmol | 0.06 | 2 | 0 | 5 h | no | 100% |
| **5g** | (4g) 0.09 mmol | 0.04 | 1 | 1 | 5 h | no | 97% |
| **5h** | (4h) 0.2 mmol | 0.04 | 3 | 3 | 5 h | no | 97% |

### N-(4-amino-2-methoxyphenyl)picolinamide (5d):

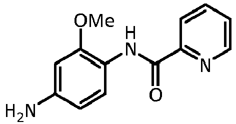

¹H NMR (400 MHz, CDCl₃) δ 10.31 (s, 1H), 8.62 (ddd, J = 4.8, 1.6, 0.9 Hz, 1H), 8.35 (d, J = 8.4 Hz, 1H), 8.27 (dt, J = 7.9, 1.0 Hz, 1H), 7.89 - 7.84 (m, 1H), 7.43 (ddd, J = 7.6, 4.8, 1.2 Hz, 1H), 6.34 (dd, J = 6.5, 4.1 Hz, 1H), 6.32 (d, J = 2.4 Hz, 1H), 3.91 (s, 3H).

### N-(4-amino-2-methoxyphenyl)nicotinamide (5e):

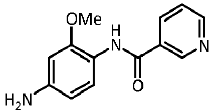

¹H NMR (400 MHz, CDCl₃) δ 9.08 (d, J = 2.4 Hz, 1H), 8.74 (dd, J = 4.8, 1.7 Hz, 1H), 8.27 (s, 1H), 8.23 - 8.15 (m, 2H), 7.42 (dd, J = 7.9, 4.8 Hz, 1H), 6.33 (dd, J = 8.5, 2.4 Hz, 1H), 6.30 (d, J = 2.4 Hz, 1H), 3.86 (s, 3H).

### N-(4-amino-2-methoxyphenyl)isonicotinamide (5f):

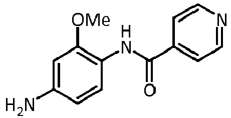

¹H NMR (400 MHz, CDCl₃). δ 8.83 - 8.72 (m, 2H), 8.31 (s, 1H), 8.21 (d, J = 8.5 Hz, 1H), 7.73 - 7.65 (m, 2H), 6.33 (dd, J = 8.5, 2.4 Hz, 1H), 6.30 (d, J = 2.4 Hz, 1H), 3.87 (s, 3H).

### N-(4-amino-2-cyanophenyl)picolinamide (5g):

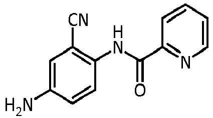

¹H NMR (400 MHz, CDCl₃) δ 8.59 - 8.53 (m, 1H), 8.18 - 8.11 (m, 1H), 8.07 (d, J = 8.9 Hz, 1H), 7.83 (td, J = 7.7, 1.6 Hz, 1H), 7.42 (ddd, J = 7.4, 4.7, 1.2 Hz, 1H), 6.88 (dd, J = 8.9, 2.7 Hz, 1H), 6.84 (d, J = 2.7 Hz, 1H).

### N-(4-aminophenyl)picolinamide (5h):

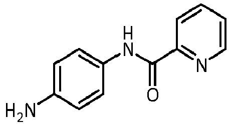

¹H NMR (400 MHz, CDCl₃) δ 9.84 (s, 1H), 8.59 (ddd, J = 4.7, 1.7, 0.9 Hz, 1H), 8.28 (dt, J = 7.9, 1.0 Hz, 1H), 7.89 (td, J = 7.7, 1.7 Hz, 1H), 7.59 - 7.54 (m, 2H), 7.45 (ddd, J = 7.5, 4.8, 1.2 Hz, 1H), 6.75 - 6.69 (m, 2H).

### Example 3. Preparation of compounds of formula 11

### Method A

To solution of 2-methoxy-4-nitroaniline **(2a)** (1.8 mmol) and di-*tert*-butyl dicarbonate (2 mmol) in DCM (5 mL), it was added TEA (4 mmol) and it was stirred 22 hours at room temperature. Afterwards, two crystals of DMAP were added and the solution was stirred at room temperature for 4 hours, additional di-*tert*-butyl dicarbonate (2 mmol) and TEA (2 mmol) were added and the resulting mixture was stirred for 22 hours at room temperature. Then, the mixture was diluted in DCM (15 ml), washed with Na₂CO₃ 1M (15 mL), saturated NaHCO₃ (15 mL), water (15 mL) and brine (15 mL), dried over anhydrous Na₂SO₄, filtered and concentrated to give a residue which was purified through silica flash column (hexane/EtOAc) to obtain **11a.** 34% yield.

### tert-Butyl (2-methoxy-4-nitrophenyl)carbamate (11a):

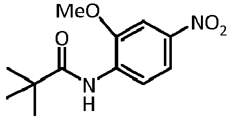

¹H NMR (400 MHz, CDCl₃) δ 8.26 (d, J =9.0 Hz, 1H), 7.90 (dd, J = 9.0, 2.4 Hz, 1H), 7.72 (d, J = 2.4 Hz, 1H), 7.35 (s, 1H), 3.98 (s, 3H), 1.54 (s, 9H).

### Method B

A dispersion of the 2-methoxy-4-nitroaniline **(2a)** (20 mmol) and phthalic anhydride (19 mmol) in AcOH (120 mL) was stirred 18 hours at reflux temperature. Afterwards, it was added 100 ml of water at room temperature. A solid precipitated and, after cooling at 0-5ºC, it was filtered off. The resulting solid was washed with cold water (5x20 ml) and it is dried under vacuum. The solid obtained was recrystallized from EtOH/EtOAc 1:1 to obtain **11b** with 54% yield.

### 2-(2-Methoxy-4-nitrophenyl)isoindoline-1,3-dione (11b):

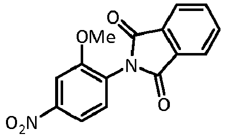

¹H NMR (400 MHz, CDCl₃) δ 8.01 - 7.95 (m, 3H), 7.91 (d, J = 2.4 Hz, 1H), 7.82 (dd, J = 5.5, 3.1 Hz, 2H), 7.46 (d, J = 8.6 Hz, 1H), 3.92 (s, 3H).

### Example 4. Preparation of compounds of formula 12.

According to the methodology shown in Example 2 method C of the preparation of compounds of general formula **5,** using the suitable compounds **11,** the following compounds **(12a)** and **(12b)** were prepared as shown in Table 5.

**Table 5**

| Prod. | Comp. 9 | mmol Pd/C | mL EtOH | mL 1,4-dioxane | Temp. | Time | Purif. | Yield |
|---|---|---|---|---|---|---|---|---|
| **12a** | (11a) 0.6 mmol | 0.2 | 4 | 0 | r.t. | 5 h | no | 98% |
| **12b** | (11b) 1.5 mmol | 0.5 | 0 | 20 | r.t. | 5 h | no | 100% |

### tert-Butyl (4-amino-2-methoxyphenyl)carbamate (12a):

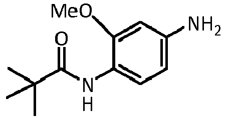

¹H NMR (400 MHz, CDCl₃) δ 7.78 (s, 1H), 6.76 (s, 1H), 6.32 (dd, J = 8.5, 2.3 Hz, 1H), 6.29 (d, J = 2.3 Hz, 1H), 3.80 (s, 3H), 1.50 (s, 9H).

### 2-(4-Amino-2-methoxyphenyl)isoindoline-1,3-dione (12b):

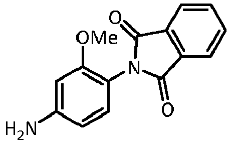

¹H NMR (400 MHz, CDCl). δ 7.97 - 7.88 (m, 2H), 7.82 - 7.74 (m, 2H), 6.98 (dd, J = 8.1, 3.4 Hz, 1H), 6.43 - 6.33 (m, 2H), 3.83 - 3.64 (m, 3H).

### Example 5. Preparation of compound of formula 13

According to the methodology shown in Example 1 method B of the preparation of compounds of general formula **4,** using the compounds **12a** and picolinic acid, the compound **13a** was prepared (Table 6).

**Table 6**

| Prod. | Comp. 10 | Comp. 3 | mmol HATU | mmol DIPEA | Solvent | Temp. | Time. | Yield |
|---|---|---|---|---|---|---|---|---|
| **13a** | (12a) 0.5 mmol | Picolinic acid 0.6 mmol | 1.1 | 1.2 | EtOAc 10 mL | 40ºC | 16 h | 88% |

### tert-Butyl (2-methoxy-4-(picolinamido)phenyl)carbamate (13a):

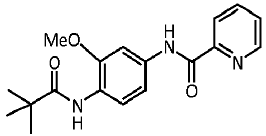

¹H NMR (400 MHz, CDCl₃) δ 9.99 (s, 1H), 8.60 (ddd, J = 4.8, 1.5, 0.9 Hz, 1H), 8.29 - 8.25 (m, 1H), 8.05 (d, J = 7.4 Hz, 1H), 7.90 (td, J = 7.7, 1.7 Hz, 1H), 7.84 (d, J = 2.1 Hz, 1H), 7.47 (ddd, J = 7.6, 4.8, 1.2 Hz, 1H), 7.02 (d, J = 6.5 Hz, 1H), 7.01 - 6.98 (m, 1H), 3.93 (s, 3H), 1.52 (s, 9H).

### Example 6. Preparation of compound 5i

To a solution of the boc-protected aniline **13a** (0.5 mmol) in DCM (9 mL), it was added TFA (3 mL) and it was stirred two hours at room temperature and it was concentrated. The obtained residue was dissolved in EtOAc (15 mL) and with Na₂CO₃ 1M (10 mL), saturated NaHCO₃ (10 mL), water (10 mL) and brine (10 mL), dried over anhydrous MgSO₄. The obtained oil was used without further purification. Quantitative yield.

### N-(4-amino-3-methoxyphenyl)picolinamide (5i):

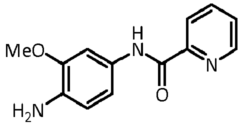

¹H NMR (400 MHz, CDCl₃) δ 9.89(s, 1H), 8.60 (d, J = 4.7 Hz, 1H), 8.27 (d, J = 7.8 Hz, 1H), 7.89 (td, J= 7.7, 1.4 Hz, 1H), 7.61 (s, 1H),7.46 (dd, J = 7.5, 4.8 Hz, 1H), 7.00(dd, J = 8.3, 2.2 Hz, 1H), 6.75 (d, J= 8.3 Hz, 1H), 3.91 (s, 3H).

### Example 7. Preparation of compound of formula 14

To solution of the aniline 12b and 2-nitrosopyridine in DCM it was added one or two drops of AcOH was stirred at room temperature. Afterwards, the solvent was evaporated under vacuum and the AcOH was coevaporated with toluene (2x5mL). The obtained solid was purified through silica *flash* column (hexane/EtOAc, DCM/MeOH or DCM/EtOAc) (see Table 7).

**Table 7**

| Prod. | Comp. 10 | mmol 2-NO-Pyr | mL DCM | Temp | Time. | Purification | Yield |
|---|---|---|---|---|---|---|---|
| **14a** | (12b) 0.9 mmol | 1.5 | 20 | r.t. | 88 h | hexane/EtOAc | 89% |

### (E)-2-(2-Methoxy-4-(pyridin-2-yldiazenyl)phenyl)isoindoline-1,3-dione (14a):

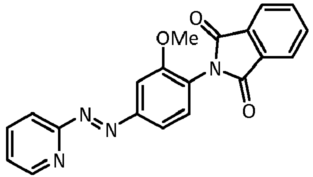

¹H NMR (400 MHz, CDCl₃) δ 8.77 (ddd, J = 4.8, 1.8, 0.9 Hz, 1H), 7.96 (dd, J = 5.5, 3.0 Hz, 2H), 7.95 -7.90 (m, 1H), 7.87 (dt, J = 8.1, 1.1 Hz, 1H), 7.84 (dd, J = 8.1, 2.0 Hz, 1H), 7.80 (dd, J = 5.5, 3.1 Hz, 2H), 7.75 (d, J = 2.0 Hz, 1H), 7.47 (d, J = 8.2 Hz,1H), 7.46 - 7.41 (m, 1H), 3.90 (s, 3H). Mass spectrum M+1= 359. RT(HPLC/DAD) = 2.96 min.

### Example 8. Preparation of compound of formula 15

To dispersion of the azocompound **14a** (0.8 mmol) in EtOH (10 mL), it was added aqueous solution of methylamine 40% (3.7 mmol) and it was stirred for two hours at 65ºC. It was concentrated and residue obtained was purified through silica *flash* column (hexane/EtOAc). 92% yield.

### (E)-2-Methoxy-4-(pyridin-2-yldiazenyl)aniline (15a):

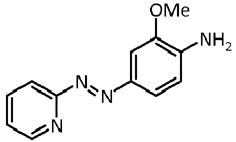

¹H NMR (400 MHz, CDCl₃) δ 8.67 (ddd, J = 4.8, 1.8, 0.9 Hz, 1H), 7.82 (ddd, J = 8.1, 7.2, 1.9 Hz, 1H), 7.75 (dt, J = 8.1, 1.1 Hz, 1H), 7.63 (dd, J = 8.2, 2.0 Hz, 1H), 7.58 (d, J = 2.0 Hz, 1H), 7.30 (ddd, J = 7.2, 4.8, 1.2 Hz, 1H), 6.76 (d, J = 8.2 Hz, 1H), 4.37 (s, 2H), 3.92 (s, 3H).

### Example 9. Preparation of compound of formula 29

According to the methodology shown in the preparation of compound of general formula **14** (see Example 7), using 4-aminobenzoic acid **(28a)**, the compound 29a was prepared (see Table 8).

**Table 8**

| Prod. | Comp. 23 | mmol 2-NO-Pyr | mL DCM | Temp. | Time | Purification | Yield |
|---|---|---|---|---|---|---|---|
| **29a** | (28a) 0.3 mmol | 0.3 | 3 | r.t. | 48 h | DCM/MeOH | 75% |

### (E)-4-(Pyridin-2-yldiazenyl)benzoic acid (29a):

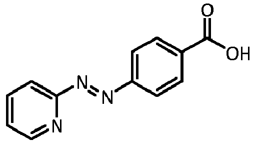

¹H NMR (400 MHz, CDCl₃). δ 8.68 (dt, J = 4.7, 1.1 Hz, 1H), 8.20 - 8.14 (m, 2H), 8.05 - 7.99 (m, 2H), 7.92 (td, J = 7.7, 1.7 Hz, 1H), 7.82 (d, J = 8.0 Hz, 1H), 7.43 (ddd, J = 7.4, 4.8, 1.3 Hz, 1H).

### Example 10. Preparation of compounds of formula 32

According to the methodology shown in the preparation of a compound of general formula **14** (see Example 7) but using 3-aminoaniline **(31a),** the compound 32a was prepared (see Table 9).

**Table 9**

| Prod. | Comp. | mmol 2-NO-Pyr | mL DCM | Temp. | Time. | Purification | Yield |
|---|---|---|---|---|---|---|---|
| **32a** | (31a) 0.5 mmol | 0.6 | 3 | r.t. | 30 h | DCM/MeOH + hexane/EtOAc | 27% |

### (E)-3-(Pyridin-2-yldiazenyl)aniline (32a):

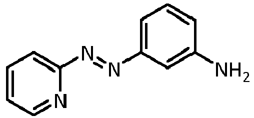

¹H NMR (400 MHz, CDCl₃) δ 8.73 (ddd, J = 4.8, 1.8, 0.8 Hz, 1H), 7.89 (ddd, J = 8.1, 7.4, 1.8 Hz, 1H), 7.80 (dt, J = 8.0, 1.0 Hz, 1H), 7.49 (ddd, J = 7.8, 1.9, 1.0 Hz, 1H), 7.39 (ddd, J = 7.3, 4.8, 1.2 Hz, 1H), 7.36 - 7.29 (m, 2H), 6.85 (ddd, J = 8.0, 2.4, 1.0 Hz, 1H), 3.84 (s, 2H).

### B. Preparation of compounds of formula 1

### Example 11. Preparation of compounds of formula 6 and 19

### Method A.

According to the methodology shown in the preparation of compound of general formula 14 (see Example 7), using the suitable compound 5 and 2-nitrosopyridine, compounds **(1a)-(1j)** were prepared as shown in Table 10.

**Table 10**

| Prod. | Comp. 5 | 2-NOPyr (mmol) | DCM (mL) | Temp. | Time. | Purification | Yield |
|---|---|---|---|---|---|---|---|
| **1a** | (5a) 0.14 mmol | 0.14 | 2 | r.t. | 35 h | DCM/EtOAc | 83% |
| **1b** | (5b) 0.14 mmol | 0.14 | 2 | r.t. | 39 h | DCM/EtOAc | 81% |
| **1c** | (5c) 0.14 mmol | 0.14 | 2 | r.t. | 75 h | DCM/EtOAc | 100% |
| **1d** | (5d) 0.12 mmol | 0.12 | 2 | r.t. | 24 h | hexane/EtOAc | 92% |
| **1e** | (5e) 0.11 mmol | 0.13 | 2 | r.t. | 64 h | hexane/EtOAc | 56% |
| **1f** | (5f) 0.13 mmol | 0.16 | 3 | r.t. | 64 h | hexane/EtOAc | 16% |
| **1g** | (5g) 0.08 mmol | 0.09 | 2 | r.t. | 40 h | hexane/EtOAc | 49% |
| **1h** | (5h) 0.2 mmol | 0.20 | 4 | r.t. | 40 h | DCM/EtOAc | 49% |
| **1i** | (5i) 0.14 mmol | 0.14 | 2 | r.t. | 25 h | hexane/EtOAc | 68% |
| **1j** | (12b) 0.9 mmol | 1.50 | 20 | r.t. | 88 h | hexane/EtOAc | 89% |

### (E)-4-Benzoyl-N-(2-methoxy-4-(pyridin-2-yldiazenyl)phenyl)benzamide (1a):

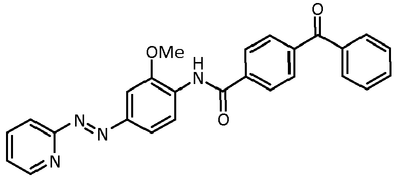

¹H NMR (400 MHz, CDCl₃) δ 8.80 (d, *J* = 4.7 Hz, 1H), 8.77 (s, 1H), 8.74 (d, *J* = 4.4 Hz, 1H), 8.02 (d, *J* = 8.2 Hz, 2H), 7.92 (d, *J* = 8.0 Hz, 2H), 7.90 - 7.83 (m, 3H), 7.82 (d, *J* = 7.4 Hz, 2H), 7.68 (s, 1H), 7.63 (t, *J* = 7.4 Hz, 1H), 7.51 (t, *J* = 7.6 Hz, 2H), 7.42 - 7.36 (m, 1H), 4.04 (s, 3H), ¹³C NMR (101 MHz, CDCl₃) δ 195.72, 164.39, 163.02, 149.49, 148.66, 148.64, 140.58, 138.33, 137.91, 136.91, 132.96, 131.52, 130.35, 130.07, 128.48, 127.11, 125.03, 123.07, 119.14, 114.51, 100.56, 56.34. Mass spectrum M+1=437. RT(HPLC/DAD)=3.75 min.

### (E)-2,4-Dichloro-N-(2-methoxy-4-(pyridin-2-yldiazenyl)phenyl)benzamide (1b):

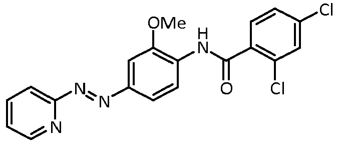

¹H NMR (400 MHz, CDCl₃) δ 8.85 (s, 1H), 8.76 (t, *J* = 5.5 Hz, 2H), 7.92 (td, *J* = 7.8, 1.8 Hz, 1H), 7.86 (dd, *J* = 7.6, 3.2 Hz, 2H), 7.79 (d, *J* = 8.3 Hz, 1H), 7.79 (d, *J* = 8.3 Hz, 1H), 7.67 (d, *J* = 1.9 Hz, 1H), 7.51 (d, *J* = 2.0 Hz, 1H), 7.44 - 7.37 (m, 2H), 3.99 (s, 3H), ¹³C NMR (101 MHz, CDCl₃) δ 163.24, 162.86, 149.33, 148.73, 138.50, 137.50, 133.27, 131.70, 131.65, 131.39, 130.38, 127.76, 125.07, 123.05, 119.33, 114.57, 102.49, 100.63, 56.28. Mass spectrum M+1=401. RT(HPLC/DAD)=3.69 min.

### (E)-2-Chloro-N-(2-methoxy-4-(pyridin-2-yldiazenyl)phenyl)benzamide (1c):

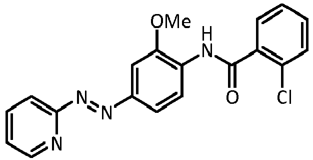

¹H NMR (400 MHz, CDCl₃) δ 8.84 (s, 1H), 8.81 (d, *J* = 8.5 Hz, 1H), 8.76 (s, 1H), 7.93 (dd, J = 11.2, 4.0 Hz, 1H), 7.89 - 7.84 (m, 2H), 7.82 (dd, *J* = 7.2, 2.1 Hz, 1H), 7.68 (d, *J* = 1.9 Hz, 1H), 7.54 - 7.37 (m, 4H), 3.99 (s, 3H), ¹³C NMR (101 MHz, CDCl₃) δ 164.33, 163.03, 149.46, 148.72, 148.65, 138.33, 134.99, 131.88, 131.56, 130.80, 130.54, 130.50, 127.29, 125.00, 123.02, 119.27, 114.51, 100.58, 56.23. Mass spectrum M+1=367. RT(HPLC/DAD)=3.33 min.

### (E)-N-(2-Methoxy-4-(pyridin-2-yldiazenyl)phenyl)picolinamide (1d):

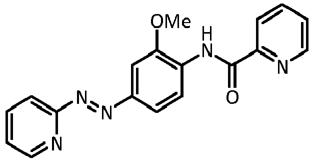

¹H NMR (400 MHz, CDCl₃) δ 10.81 (s, 1H), 8.85 (d, J = 8.5 Hz, 1H), 8.78 - 8.70 (m, 1H), 8.67 (dd, J = 4.7, 0.9 Hz, 1H), 8.31 (dd, J = 7.8, 0.9 Hz, 1H), 7.94 - 7.87 (m, 2H), 7.87-7.81 (m, 2H), 7.68 (d, J = 1.9 Hz, 1H), 7.49 (ddd, J = 7.5, 4.8, 1.2 Hz, 1H), 7.39 (ddd, J = 7.2, 4.8, 1.2 Hz, 1H), 4.07 (s, 3H).¹³C NMR (101 MHz, CDCl₃) δ 163.14, 162.30, 149.87, 149.46, 149.31, 148.55, 148.27, 138.28, 137.60, 131.69, 126.55, 124.89, 123.03, 122.50, 118.90, 114.44, 100.61, 56.25. Mass spectrum M+1=334. RT(HPLC/DAD)=3.66 min.

### (E)-N-(2-Methoxy-4-(pyridin-2-yldiazenyl)phenyl)nicotinamide (1e):

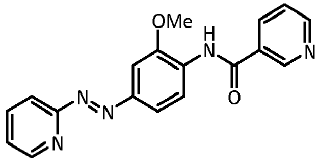

¹H NMR (400 MHz, CDCl₃). δ 9.16 (dd, J = 2.4, 0.8 Hz, 1H), 8.81 (dd, J = 4.9, 1.7 Hz, 1H), 8.76 (d, J = 8.6 Hz, 3H), 8.26 (ddd, J = 7.9, 2.3, 1.7 Hz, 1H), 7.95 - 7.81 (m, 3H), 7.69 (d, J = 2.1 Hz, 1H), 7.49 (ddd, J = 8.0, 4.8, 0.9 Hz, 1H), 7.41 (ddd, J = 7.2, 4.8, 1.2 Hz, 1H), 4.05 (s, 3H). Mass spectrum M+1=334. RT(HPLC/DAD)= 2.92 min.

### (E)-N-(2-Methoxy-4-(pyridin-2-yldiazenyl)phenyl)isonicotinamide (1f):

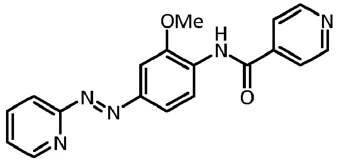

¹H NMR (400 MHz, CDCl₃). δ 8.85 (dd, J = 4.4, 1.6 Hz, 2H), 8.78 - 8.73 (m, 3H), 7.96 - 7.82 (m, 3H), 7.76 (dd, J = 4.4, 1.6 Hz, 2H), 7.69 (d, J = 2.0 Hz, 1H), 7.42 (ddd, J = 7.3, 4.8, 1.2 Hz, 1H), 4.05 (s, 3H). ¹³C NMR (101 MHz, CDCl₃ Mass spectrum M+1=334. 2.242. RT(HPLC/DAD) = 2.95 min.

### (E)-N-(2-Cyano-4-(pyridin-2-yldiazenyl)phenyl)picolinamide (1g):

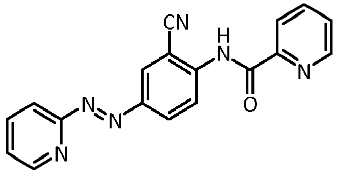

¹H NMR (400 MHz, CDCl₃) δ 11.09 (s, 1H), 8.95 (d, J = 9.8 Hz, 1H), 8.75 (ddd, J = 4.8, 1.8, 0.8 Hz, 1H), 8.72 (ddd, J = 4.7, 1.7, 0.9 Hz, 1H), 8.38 - 8.33 (m, 2H), 8.30 (dt, J = 7.8, 1.1 Hz, 1H), 7.97 - 7.90 (m, 2H), 7.85 (dt, J = 8.0, 1.0 Hz, 1H), 7.55 (ddd, J = 7.6, 4.8, 1.2 Hz, 1H), 7.43 (ddd, J = 7.4, 4.8, 1.2 Hz, 1H). Mass spectrum M+1= 329. RT(HPLC/DAD) = 3.37 min.

### (E)-N-(4-(Pyridin-2-yldiazenyl)phenyl)picolinamide (1h):

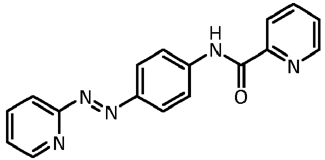

¹H NMR (400 MHz, CDCl₃) δ 10.29 (s, 1H), 8.74 (ddd, J = 4.8, 1.9, 0.9 Hz, 1H), 8.64 (ddd, J = 4.8, 1.7, 0.9 Hz, 1H), 8.32 (dt, J = 7.8, 1.1 Hz, 1H), 8.16 - 8.10 (m, 2H), 8.01 - 7.97 (m, 2H), 7.96 - 7.88 (m, 2H), 7.83 (dt, J = 8.0, 1.1 Hz, 1H), 7.51 (ddd, J = 7.6, 4.8, 1.2 Hz, 1H), 7.39 (ddd, J = 7.3, 4.7, 1.2 Hz, 1H). Mass spectrum M+1= 304. RT(HPLC/DAD) = 3.16 min.

### (E)-N-(3-Methoxy-4-(pyridin-2-yldiazenyl)phenyl)picolinamide (1i):

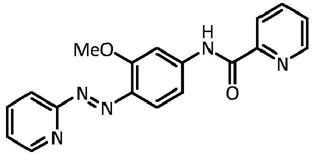

¹H NMR (400 MHz, CDCl₃) δ 10.29 (s,1H), 8.70 (dd, J = 4.7, 1.3 Hz, 1H), 8.63 (d, J = 4.7 Hz, 1H), 8.30 (d, J = 7.8 Hz, 1H), 8.09 (d, J = 2.1 Hz, 1H), 7.97 - 7.91 (m, 2H), 7.87 - 7.83 (m, 1H), 7.78 (d, J = 8.0 Hz, 1H), 7.51 (ddd, J = 7.6, 4.8, 1.1 Hz, 1H), 7.34 (ddd, J = 7.2, 4.8, 1.1 Hz, 1H), 7.11 (dd, J = 8.8, 2.1 Hz, 1H), 4.11(s, 3H). ¹³C NMR (101 MHz, CDCl₃) δ 163.69, 162.28, 159.14, 149.40, 149.24, 148.07, 143.16, 138.28, 138.14, 137.86, 126.83, 124.55, 122.45, 117.93, 114.57, 111.64, 103.27, 56.30. Mass spectrum M+1=334. RT(HPLC/DAD)=3.07 min.

### (E)-2-(2-Methoxy-4-(pyridin-2-yldiazenyl)phenyl)isoindoline-1,3-dione (1j):

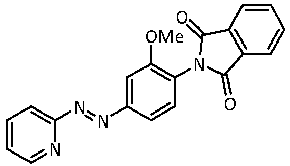

¹H NMR (400 MHz, CDCl₃) δ 8.77 (ddd, J = 4.8, 1.8, 0.9 Hz, 1H), 7.96 (dd, J = 5.5, 3.0 Hz, 2H), 7.95 -7.90 (m, 1H), 7.87 (dt, J = 8.1, 1.1 Hz, 1H), 7.84 (dd, J = 8.1, 2.0 Hz, 1H), 7.80 (dd, J = 5.5, 3.1 Hz, 2H), 7.75 (d, J = 2.0 Hz, 1H), 7.47 (d, J = 8.2 Hz,1H), 7.46 - 7.41 (m, 1H), 3.90 (s, 3H). Mass spectrum M+1= 359. RT(HPLC/DAD) = 2.96 min.

### Method B

According to the methodology shown in method A of the general preparation of compounds of general formula **4** (see Example 1), using the amine **15a** and the suitable acid chlorides **3** or isocyanates **15,** the following compounds **(1k)-(1o)** were prepared as shown in Table 11.

**Table 11**

| Prod. | mmol comp. (13a) | Comp. 3 or 14 | mmol DIPEA | solvent | Temp. | Time | Purif. | Yield |
|---|---|---|---|---|---|---|---|---|
| **1k** | 0.08 | Benzoyl chloride 0.18 mmol | 0.3 | DCE 1 mL | 40ºC | 2 h | yes* | 71% |
| **1l** | 0.08 | Benzoyl chloride 0.18 mmol | 0.3 | DCE 1 mL | 40ºC | 2 h | yes* | 17% |
| **1m** | 0.13 | Isobutyryl chloride 0.14 mmol | 0.3 | DCE 1.5 mL | 40ºC | h | no | 100% |
| **1n** | 0.13 | Cyclohexanecarbonyl chloride 0.14 mmol | 6.4 | DCE 1.5 mL | 100ºC | 1 h | no | 100% |
| **1o** | 0.13 | 1-fluoro-2-isocyanatebenzene 0.14 mmol | 0.14 | THF 1.5 mL | 100ºC | 1 h | yes** | 100% |

| | | | | | | | | |
|---|---|---|---|---|---|---|---|---|
| * Compounds (1k) and (11) were purified with silica column (DCM/MeOH), C18 column (water/MeCN), finally, with preparative HPLC (water/MeOH). ** Compound (1o) was purified through C18 column (water/MeCN). | | | | | | | | |

### (E)-N-Benzoyl-N-(2-methoxy-4-(pyridin-2-yldiazenyl)phenyl)benzamide (1k):

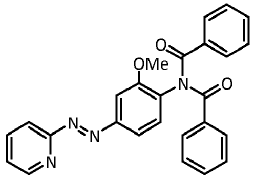

¹H NMR (400 MHz, CDCl₃) δ 8.78 - 8.72 (m, 1H), 7.91 (td, J = 7.7, 1.9 Hz, 1H), 7.83 - 7.79 (m, 1H), 7.76 (dd, J = 8.2, 1.4 Hz, 4H), 7.68 - 7.62 (m, 2H), 7.47 - 7.40 (m, 3H), 7.33 (dd, J = 8.4, 6.9 Hz, 4H), 7.26 - 7.22 (m, 1H), 3.90 (s, 3H). Mass spectrum M+1= 437. RT(HPLC/DAD) = 3.14 min.

### (E)-N-(2-Methoxy-4-(pyridin-2-yldiazenyl)phenyl)benzamide (1l)

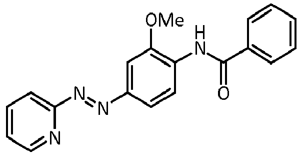

¹H NMR (400 MHz, CDCl₃) δ 8.79 (d, J = 8.6 Hz, 1H), 8.77 (s, 1H), 8.75 (dd, J = 4.7, 1.8 Hz, 1H), 7.97 - 7.81 (m, 5H), 7.68 (d, J = 2.0 Hz, 1H), 7.60 - 7.50 (m, 3H), 7.40 (ddd, J = 7.2, 4.8, 1.2 Hz, 1H), 4.04 (s, 3H). Mass spectrum M+1= 333. RT(HPLC/DAD) = 3.40 min.

### (E)-N-(2-Methoxy-4-(pyridin-2-yldiazenyl)phenyl)isobutyramide (1m):

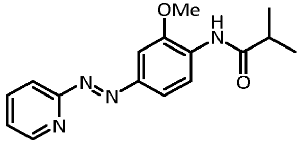

¹H NMR (400 MHz, CDCl₃) δ 8.72 (ddd, J = 4.9, 1.8, 0.8 Hz, 1H), 8.64 (d, J = 8.6 Hz, 1H), 8.02 (s, 1H), 7.87 (ddd, J = 8.2, 7.3, 1.9 Hz, 1H), 7.82 - 7.75 (m, 2H), 7.61 (d, J = 2.1 Hz, 1H), 7.37 (ddd, J = 7.2, 4.8, 1.2 Hz, 1H), 3.97 (s, 3H), 2.60 (hept, J = 6.9 Hz, 1H), 1.28 (d, J = 6.9 Hz, 6H). Mass spectrum M+1= 299. RT(HPLC/DAD) = 3.00 min.

### (E)-N-(2-Methoxy-4-(pyridin-2-yldiazenyl)phenyl)cyclohexanecarboxamide (1n):

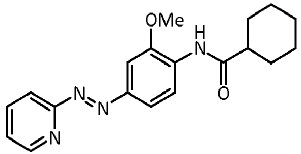

¹H NMR (400 MHz, CDCl₃) δ 8.71 (ddd, J = 4.8, 1.8, 0.9 Hz, 1H), 8.63 (d, J = 8.6 Hz, 1H), 8.01 (s, 1H), 7.87 (ddd, J = 8.1, 7.3, 1.8 Hz, 1H), 7.81 - 7.73 (m, 2H), 7.60 (d, J = 2.1 Hz, 1H), 7.36 (ddd, J = 7.2, 4.8, 1.2 Hz, 1H), 3.97 (s, 3H), 2.35 - 2.25 (m, 1H), 1.98 (dtt, J = 13.4, 3.5, 1.7 Hz, 2H), 1.84 (dt, J = 12.9, 3.4 Hz, 2H), 1.60 (d, J = 8.5 Hz, 1H), 1.58 - 1.48 (m, 1H), 1.37 - 1.19 (m, 4H). Mass spectrum M+1= 339. RT(HPLC/DAD) = 3.56 min.

### (E)-1-(2-Fluorophenyl)-3-(2-methoxy-4-(pyridin-2-yldiazenyl)phenyl)urea (1o):

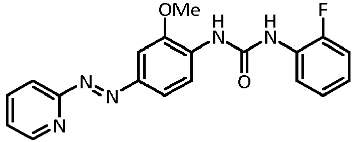

¹H NMR (400 MHz, CDCl₃) δ 8.72 (ddd, J = 4.8, 1.9, 0.9 Hz, 1H), 8.46 (d, J = 8.6 Hz, 1H), 8.22 - 8.11 (m, 1H), 7.89 (ddd, J = 8.1, 7.3, 1.9 Hz, 1H), 7.82 (dt, J = 8.1, 1.1 Hz, 1H), 7.77 (dd, J = 8.6, 2.1 Hz, 1H), 7.56 (d, J = 2.1 Hz, 1H), 7.38 (ddd, J = 7.3, 4.8, 1.2 Hz, 1H), 7.16 - 6.95 (m, 5H), 3.85 (s, 3H). Mass spectrum M+1=366. RT(HPLC/DAD) = 3.58 min.

### Method C

According to the methodology shown in Example **1** method B of the preparation of compounds of general formula **4,** using the amine **15a** and the suitable compounds **3,** the following compounds of general formula **(1p-1sr)** were prepared as shown in Table 12.

**Table 12**

| Prod. | mmol Comp. 13a | Comp.3 | mmol HATU | mmol DIPEA | Solvent | Temp. | Time | Yield |
|---|---|---|---|---|---|---|---|---|
| **1p*** | 0.13 | 2-fluorobenzoic acid 0.2 mmol | 0.5 | 0.5 | DMF 1.5 mL | 40ºC | 22 h | 52% |
| **1q*** | 0.13 | 2-cyanobenzoic acid 0.2 mmol | 0.5 | 0.5 | DMF 1.5 mL | 40ºC | 22h | 72% |
| **1r**** | 0.13 | thiazole-4-carboxilic acid 0.2 mmol | 0.4 | 0.4 | DMF 1.5 mL | 40ºC | 22 h | 68% |

| | | | | | | | | |
|---|---|---|---|---|---|---|---|---|
| * Compounds (1q) and (1r) were purified with silica column (DCM/MeOH). ** Compound (1s) was purified through C18 column (water/MeCN) | | | | | | | | |

### (E)-2-Fluoro-N-(2-methoxy-4-(pyridin-2-yldiazenyl)phenyl)benzamide (1p):

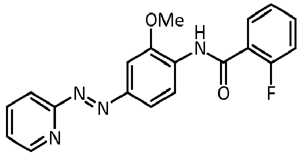

¹H NMR (400 MHz, CDCl₃) δ 9.46 (d, J = 15.7 Hz, 1H), 8.80 (d, J = 8.6 Hz, 1H), 8.73 (ddd, J = 4.8, 1.9, 0.9 Hz, 1H), 8.19 (td, J = 7.9, 1.9 Hz, 1H), 7.89 (ddd, J = 8.1, 7.2, 1.8 Hz, 1H), 7.85 - 7.79 (m, 2H), 7.66 (d, J = 2.0 Hz, 1H), 7.57 - 7.48 (m, 1H), 7.38 (ddd, J = 7.2, 4.8, 1.2 Hz, 1H), 7.35 - 7.29 (m, 1H), 7.19 (ddd, J = 12.2, 8.3, 1.1 Hz, 1H), 4.02 (s, 3H). Mass spectrum M+1= 351. RT(HPLC/DAD) = 3.85 min.

### (E)-2-Cyano-N-(2-cyanobenzoyl)-N-(2-methoxy-4-(pyridin-2-yldiazenyl)phenyl) benzamide (1q):

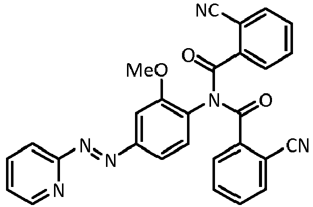

¹H NMR (400 MHz, CDCl₃) δ 8.77 (ddd, J = 4.7, 1.9, 0.9 Hz, 1H), 8.01 - 7.85 (m, 5H), 7.80 (dd, J = 8.2, 2.0 Hz, 1H), 7.78 - 7.73 (m, 3H), 7.65 - 7.56 (m, 3H), 7.54 (d, J = 8.2 Hz, 1H), 7.45 (ddd, J = 7.3, 4.8, 1.2 Hz, 1H), 3.72 (s, 3H). Mass spectrum M+1= 487. RT(HPLC/DAD) = 3.03 min.

### (E)-N-(2-Methoxy-4-(pyridin-2-yldiazenyl)phenyl)thiazole-4-carboxamide (1r):

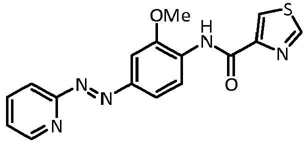

¹H NMR (400 MHz, CDCl₃) δ 10.07 (s, 1H), 8.83 (d, J = 2.1 Hz, 1H), 8.77 (d, J = 8.6 Hz, 1H), 8.72 (ddd, J = 4.8, 1.9, 0.9 Hz, 1H), 8.29 (d, J = 2.1 Hz, 1H), 7.88 (ddd, J = 8.1, 7.3, 1.8 Hz, 1H), 7.84 - 7.79 (m, 2H), 7.66 (d, J = 2.0 Hz, 1H), 7.37 (ddd, J = 7.2, 4.7, 1.2 Hz, 1H), 4.03 (s, 3H). Mass spectrum M+1=340. RT(HPLC/DAD) = 3.43 min.

### Example 12. Preparation of compounds of formula 30

A dispersion of carboxylic acid 29a (0.09 mmol) in thionyl chloride (1 mL) was stirred at 75ºC for 3.5 hours. Then, it was evaporated to dryness and dissolved in DCE (2 mL) with 2-aminopyridine (0.2mmol) and DIPEA (0.4 mmol). The resulting solution was stirred 3 hours at 90ºC in a suitable reactor and 64 hours at room temperature. Afterwards the solution was concentrated, redissolved in EtOAc (15 mL), washed with Na₂CO₃ 1M (10 mL), saturated NaHCO₃ (10 mL), water (10 mL) and brine (10 mL), dried over anhydrous Na₂SO₄, filtered and concentrated to give a residue which was purified twice through silica flash column (hexane/EtOAc) and (DCM/MeOH). 26% yield.

### (E)-N-(Pyridin-2-yl)-4-(pyridin-2-yldiazenyl)benzamide (1s):

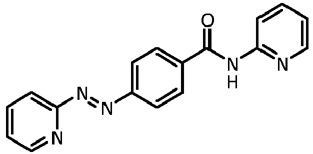

¹H NMR (400 MHz, CDCl₃) δ 8.76 (ddd, J = 4.9, 1.9, 0.9 Hz, 1H), 8.74 (s, 1H), 8.39 (dt, J = 8.3, 1.0 Hz, 1H), 8.29 (ddd, J = 4.9, 1.9, 0.9 Hz, 1H), 8.17 - 8.11 (m, 2H), 8.11 - 8.07 (m, 2H), 7.94 (ddd, J = 8.0, 7.3, 1.8 Hz, 1H), 7.87 (dt, J = 8.0, 1.0 Hz, 1H), 7.77 (ddd, J = 8.4, 7.4, 1.9 Hz, 1H), 7.44 (ddd, J = 7.3, 4.7, 1.2 Hz, 1H), 7.08 (ddd, J = 7.3, 4.9, 1.0 Hz, 1H). Mass spectrum M+1= 304. RT(HPLC/DAD) = 2.89 min.

### Example 13. Preparation of compounds of formula 33

According to the methodology shown in Example 2 method A, using the amine **32a** and the suitable compound **3,** compound **1t** was prepared as shown in Table 13.

**Table 13**

| Prod. | Comp. 32a | Comp. 3 | mmol HATU | mmol DIPEA | Solvent | Temp. | Time. | Yield |
|---|---|---|---|---|---|---|---|---|
| **1t** | 0.09 mmol | Picolinic acid 0.13 mmol | 0.3 | 0.3 | DMF 1 mL | 40ºC | 16 h | 84% |

### (E)-N-(3-(pyridin-2-yldiazenyl)phenyl)picolinamide (1t):

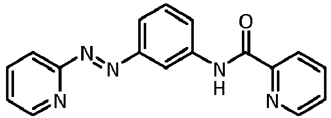

¹H NMR (400 MHz, CDCl₃) δ 10.23 (s, 1H), 8.76 (ddd, J = 4.7, 1.8, 0.9 Hz, 1H), 8.64 (dt, J = 4.7, 1.5 Hz, 1H), 8.37 - 8.26 (m, 2H), 8.15 (ddd, J = 8.1, 2.3, 1.0 Hz, 1H), 7.93 (td, J = 7.5, 1.8 Hz, 2H), 7.87 (ddt, J = 9.1, 7.9, 1.1 Hz, 2H), 7.58 (t, J = 8.0 Hz, 1H), 7.50 (ddd, J = 7.6, 4.8, 1.2 Hz, 1H), 7.42 (ddd, J = 7.3, 4.8, 1.2 Hz, 1H). Mass spectrum M+1= 304. RT(HPLC/DAD)=3.12 min.

The (Z) isomer of the compounds of formula **(1a-1t)** can be obtained by irradiation at 380 nm of the corresponding (*E*) isomer. Exceptionally, **(*Z*)-1h** can be obtained by irradiation between 425 and 475 nm of the corresponding (*E*) isomer and **(*Z*)-1p** by irradiation from 380 nm to 455 nm of the corresponding (*E*) isomer.

### C. Synthesis of compounds for comparative Examples

### Preparation of compound 44

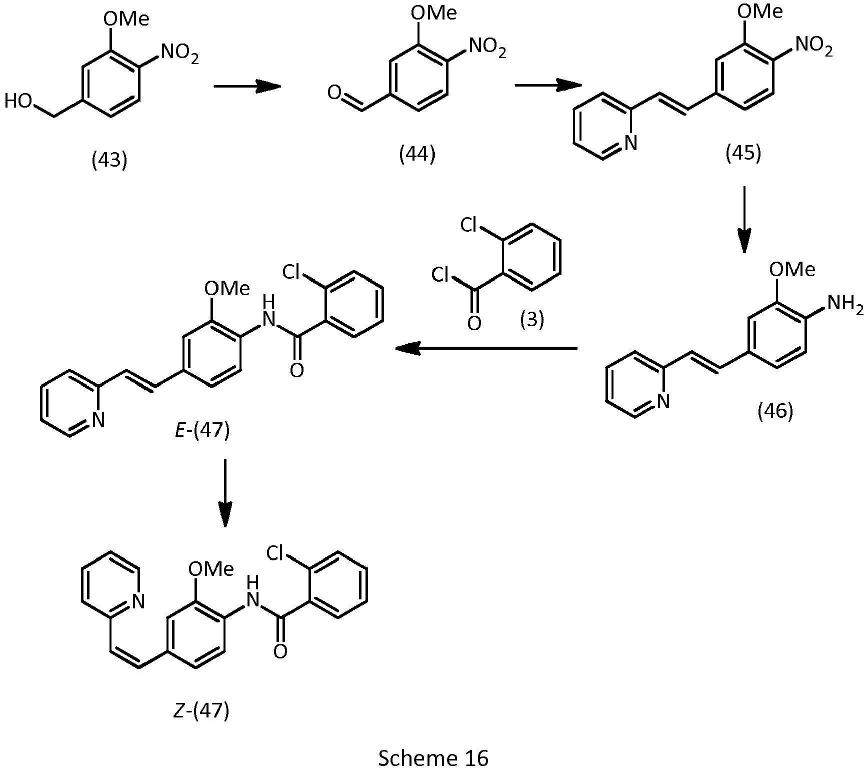

**3-Methoxy-4-nitrobenzaldehyde (44):** A dispersion of (3-methoxy-4-nitrophenyl)methanol **(43)** (1.4 mmol) and pyridinium dichromate (2.3 mmol) in dry DCM(38 mL) was stirred 17 hours at room temperature and a mixture of celite and silica was added. The dispersion was filtered and washed with DCM (3x10 mL). The solvent of the resulting solution was evaporated under vacuum to give a brown residue (1.3 mmol, quantitative yield). ¹H NMR (400 MHz, CDCl₃) δ 10.05 (s, 1H), 7.92 (d, J = 8.1 Hz, 1H), 7.60 (d, J = 1.3 Hz, 1H), 7.54 (dd, J = 8.1, 1.5 Hz, 1H), 4.03 (s, 3H).

**2-(3-Methoxy-4-nitrostyryl)pyridine (45):** A dispersion of 3-methoxy-4-nitrobenzaldehyde **(44)** (1.1 mmol) and 2-picoline (4.1 mmol) in acetic anhydride (4 mL) was stirred 38 hours at 130ºC and was diluted with methanol (15 mL), the solvent was evaporated under vacuum and coevaporated with toluene (2x10 mL). The obtained residue was dissolved in EtOAc (50 mL) and was washed with aqueous NaOH 2M (2x15 mL). The solvent was removed under vacuum to give a black solid, which was purified through silica flash column (hexane/EtOAc) to give thick oil that crystallises when chilling (0.40 mmol, 38% yield). ¹H NMR (400 MHz, CDCl₃) δ 8.64 (d, J = 4.1 Hz, 1H), 7.91 (d, J = 8.8 Hz, 1H), 7.72 (td, J = 7.7, 1.8 Hz, 1H), 7.64 (d, J = 16.0 Hz, 1H), 7.42 (d, J = 7.8 Hz, 1H), 7.29 - 7.20 (m, 4H), 4.01 (s, 3H).

**2-Methoxy-4-(2-(pyridin-2-yl)vinyl)aniline (46): A** dispersion of 2-(3-methoxy-4-nitrostyryl)pyridine (45) (0.4 mmol) and sodium sulfide nonahydrate (1.8 mmol) in ethanol (13 mL) was stirred one hours at reflux temperature, it was poured into water (80 mL) and extracted with EtOAc (3x30 mL). The joined organic layers were dried over anhydrous MgSO₄, filtered off and the solvent was removed under vacuum to give dark orange oil (0.38 mmol, 90% yield). ¹H NMR (400 MHz, CDCl₃) δ 8.56 (ddd, J = 4.8, 1.7, 0.8 Hz, 1H), 7.63( td, J = 7.7, 1.8 Hz, 1H), 7.52 (d, J = 16.1 Hz, 1H), 7.36 (d, J = 7.9 Hz, 1H), 7.11 - 7.07 (m, 1H), 7.07 (d, J = 1.7 Hz, 1H), 7.05 - 6.96 (m, 2H), 6.69 (d, J = 8.0 Hz, 1H), 3.90 (s, 3H).

**(*E*)- and (*Z*)-2-Chloro-*N*-(2-methoxy-4-(2-(pyridin-2-yl)vinyl)phenyl)benzamide (47):** A dispersion of 2-chlorobenzoic acid (0.33 mmol) in thionyl chloride (0.8 mL) was heated for two hours at 75ºC and the solvent was removed under vacuum and coevaporated with toluene (2x3 mL) to give a colourless oil which was dissolved with 2-methoxy-4-(2-(pyridin-2-yl)vinyl)aniline (46) (0.30 mmol) in DCE (3 mL) and it was added DIPEA (1.6 mmol) and it was stirred at 90ºC for one hour and concentrated. The resulting residue is dissolved in EtOAc (40 mL) and washed with Na₂CO₃ 1N (15 mL), water (15 mL) and brine (15 mL), dried over anhydrous MgSO₄ and the solvent is removed under vacuum to give a residue which was purified through silica flash column (hexane/EtOAc) to give thick oil that crystallises when chilling. This solid corresponds to 98% **(E)-47** and 2% **(Z)-47** (0.24 mmol, 80% yield). A part of the solid (0.07 mmol) is dissolved in chloroform (0.7 mL) and it is left under natural light 23 days and the solvent was removed under vacuum to give a residue, which was separated through C18 flash column (water/methanol) to give **(Z)-47** (0.007 mmol, 10% yield) and **(E)-47** (0.03 mmol, 38% yield).

**(E)-2-Chloro-N-(2-methoxy-4-(2-(pyridin-2-yl)vinyl)phenyl)benzamide ((E)-47):** ¹H NMR (400 MHz, CDCl₃) δ 8.64 (s, 1H), 8.61 - 8.59 (m, 1H), 8.57 (d, J = 8.3 Hz, 1H), 7.79 (dd, J = 7.1, 2.2 Hz, 1H), 7.67 (td, J = 7.7,1.8 Hz, 1H), 7.59 (d, J = 16.1 Hz, 1H), 7.47 (dd, J = 7.7, 1.6 Hz, 1H), 7.45 - 7.35 (m, 3H), 7.26 - 7.22 (m, 1H), 7.18 - 7.10 (m, 3H), 3.94 (s, 3H). RT(HPLC/DAD)=3.35 min.

**(*Z*)-2-Chloro-*N*-(2-methoxy-4-(2-(pyridin-2-yl)vinyl)phenyl)benzamide ((*Z*)-47):** ¹H NMR (400 MHz, CDCl₃) δ 8.59 (d, *J* = 4.1 Hz, 1H), 8.55 (s, 1H), 8.43(d, J=8.3Hz, 1H), 7.75 (dd, *J* = 7.1, 2.2 Hz, 1H), 7.52 - 7.33 (m, 4H), 7.26 - 7.24 (m, 1H), 7.11 - 7.06 (m, 1H), 6.94 (d, *J* = 8.3 Hz, 1H), 6.91 (d, *J* = 1.7 Hz, 1H), 6.76 (d, *J* = 12.4 Hz, 1H), 6.65 (d, *J* = 12.4 Hz, 1H), 3.67 (s, 3H). RT(HPLC/DAD)=3.12 min.

### Pharmacological experiments

Pharmacological experiments on mGlu receptors were performed in HEK293 cells cultured in Dulbecco's modified Eagle's medium (GIBCO-BRL Life Technologies Inc., Cergy Pontoise, France) supplemented with 10% fetal calf serum (FCS, GIBCO-BRL Life Technologies., Cergy Pontoise, France). The compounds were tested on a rat mGlu5 receptor, which was transiently transfected in HEK293 cells by electroporation as described elsewhere *[*Neuropharmacology 1998, 37, 1043-1051]. Additionally, a plasmid encoding the high-affinity glutamate transporter EAAC1 was cotransfected to prevent the influence of the glutamate released by the cells in the medium. Receptor activity is monitored by measurement of IP1 production using the kit IP-One HTRF^{®} Assay from Cisbio Bioassays (France). For IP1 accumulation measurements, cells were seeded after transfection in polyornithine-coated, black-walled 96-well Polystyrene Cell Culture Microplates (Greiner Bio-One SAS, France) and cultured for 24 h in glutamate/glutamine-free medium (GIBCO-BRL Life Technologies Inc., Cergy Pontoise, France) and antibiotics (penicillin and streptomycin, 100 UmL_1 final, GIBCO-BRL Life Technologies). Media was removed and cells were incubated for 30min in a solution containing the drugs at the tested concentrations. The IP-One stimulation buffer, provided with the kit IP-One (Cisbio Bioassays, France), contains lithium chloride (LiCl) at 50mM that inhibits the degradation of IP1 to Myo-inositol, which is then accumulated in the cell. HTRF reagents, monoclonal antibody specific to IP1 labelled with terbium cryptate and IP1 coupled with the dye d2, diluted in a Lysis buffer were added. After 1 hour of incubation, time resolved fluorescence at 620nm and 665nm was measured with RUBYstar (BMG Labtech, Germany). The specific signal, which results from the energy transfer between europium cryptate and the donor d2, is proportional to the ratio between the fluorescence emitted at 620 nm and 665 nm, and consequently to the concentration of IP1 in the lysate. All points were determined in triplicate. The dose-response curves were fitted using GraphPad Prism software and the following equation: y= [(yₘₐₓ-yₘᵢₙ)/(1+10^(LogIC50-x))]+yₘᵢₙ, in which IC50 is the concentration of the compound necessary to obtain the half-maximal effect. The results are shown in Table 14. (10 µM ≤ IC50 < 50 µM + ; 2 µM ≤ IC50 < 10 µM ++ ; 500 nM ≤ IC₅₀ < 2 µM +++; 100 nM ≤ IC₅₀<500 nM ++++; IC₅₀< 100 nM +++++)

**Table 14.**

| Compound | IC₅₀ mGlu5 + 100 nM Quisqualate |
|---|---|
| 1a | +++ |
| 1b | +++ |
| 1c | +++++ |
| 1d | ++++ |
| 1e | ++++ |
| 1f | +++ |
| 1g | ++ |
| 1h | ++ |
| 1i | + |
| 1j | ++ |
| 1k | +++ |
| 1l | ++++ |
| 1m | +++++ |
| 1n | +++++ |
| 1o | +++ |
| 1p | ++++ |
| 1r | ++++ |
| 1s | + |
| 1t | + |

### Experiments of mGlu5 responses

### Materials and methods

*Cell Culture and Transient Transfection:* Human embryonic kidney (HEK) tsA201 cells were maintained at 37ºC and humidified atmosphere with 5% CO₂; cells were grown in Dulbecco's Modified Eagle's Medium/Nutrient Mixture F-12 Ham (DMEM/F12, 1:1) medium, supplemented with 10% Fetal Bovine Serum (FBS) and antibiotics (1% Penicillin/Streptomycin). Once at confluence, culture flasks were washed twice with PBS and harvested with 300 µl Accutase. Expression of rat mGlu5a was induced by transient transfection of the cDNA-containing plasmid with X-tremeGENE 9 DNA Transfection Reagent. For transfection, cells were seeded at a concentration of 10⁶ cells per well on 6-multiwell plates with a final volume of 2ml, and immediately transfected following manufacturer's instructions (100 µl mixture of 3:1 (µl:µg) of X-tremeGENE 9 DNA TR:mGlu5a plasmid in serum-free medium, 20 minutes incubation). About 24h after transfection, HEK tsA201 cells transiently overexpressing mGlu5a were seeded onto 15 mm glass coverslips pre-treated with Poly-L-Lysine to allow cell adhesion. Seeding concentration was maintained between 2/4·10⁵ cells to get preconfluent cultures at the time of experiment. Transfected cells were used for single-cell calcium imaging experiments between 48 and 72 hours after transfection.

*Single-cell Calcium Imaging:* At the time of experiments, glass coverslips were mounted onto the recording chamber, and cells were loaded with the high affinity, ratiometric, intracellular calcium indicator Fura-2 AM; the dye was incubated during 30 minutes at 37°C and 5% CO₂, at a final concentration of 10 µM in Ca²⁺-free extracellular solution (ES, -Ca²⁺). We used a ratiometric calcium sensor in order to avoid bleaching artefacts. Cells were then carefully rinsed with fresh ES, three to four times, before the recording chamber was placed on an inverted fully-motorised digital microscope (iMic 2000, Till Photonics, Gräfelfing, Germany). Images were acquired at room temperature with a UV Apochromat 40x oil objective lens (Olympus, Tokio, Japan). Fura-2 AM was excited consecutively for 10 ms at two different wavelengths (340 and 380 nm) as indicated by the manufacturer (Life Technologies, Paisley, UK) by using a Polychrome V light source (Till Photonics) and a 505 nm dichroic beamsplitter (Chroma Technology, Below Falls, VT, USA); emission at 510 nm was filtered by a D535/40nm emission filter (Chroma Technology) and finally collected by a cooled CCD camera (Interline Transfer IMAGO QE, Till Photonics). The fluorescence measurements (excitation + emission recording) were carried out every 2 seconds, and each measurement takes about 10-50 ms. The 340 and 380 nm images were stored by the TILLvisION imaging software (Till Photonics); the ratio of the emission when the sample was excited at 340 nm and the emission when it was excited at 380 nm was recorded and its mean value was calculated for each cell using the same software. Higher ratios correspond to higher intracellular calcium concentrations.

Agonists and allosteric modulators addition was obtained by carefully pipetting an initial 1:30 dilution of the compound directly into the accessory pool of the recording chamber, obtaining a final dilution of 1:1000 (total DMSO concentration was always lower than 1%). Activation of the agonist was induced in HEK tsA201 mGlu5a transfected cells with the glutamate analogue Quisqualic acid (3 µM). Only evoked, sustained intracellular calcium oscillations were taken into account as consistent cell responses to the application of the agonist; different responses (such as single-peak, peak-and-plateau, etc.), as well as spontaneously oscillating cells, were discarded as unsuitable for calculating the oscillatory frequency.

*Solutions and Reagents:* The extracellular solution (ES) used for single-cell calcium imaging contained: 140 mM NaCl, 5.4 mM KCI, 1 mM MgCl₂, 10 mM HEPES, 10 mM glucose and 2 mM CaCl₂ adjusted to pH 7.4 with NaOH. Agonists and allosteric compounds were stored at -20ºC in DMSO, and dissolved in ES at the time of experiments. Reagents were obtained as follows: DMEM/F12, FBS, Fura-2 AM (Life Technologies, Paisley, UK); X-tremeGENE 9 Transfection Reagent (Roche Applied Science); SIB-1757 (Sigma-Aldrich); VU-29 (Tocris Bioscience, Bristol, UK); penicillin/streptomycin, Accutase, Poly-L-Lysine, Quisqualic Acid, MPEP and all other chemicals (Sigma-Aldrich, St. Louis, MO, USA).

### Activity results

Activation of mGlu5 by orthosteric ligands, such as quisqualate, results in strong, sustained intracellular calcium oscillations. Allosteric modulators can change this response by reducing or increasing the oscillatory frequency.

We explored the biological activity of the compounds by single-cell calcium imaging on HEK tsA201 cells transiently expressing the rat metabotropic glutamate receptor 5 (mGlu5). This technique allows evaluating cell by cell the changes in the intracellular calcium concentration. Receptor activation was triggered by adding 3 µM quisqualate into the recording chamber. Secondly, compounds were added, still in the presence of the agonist, and monitored how the different molecules influenced the cell response. A reduction of the frequency of intracellular calcium was observed when Compounds 1 were added to HEK tsA201 cells transiently expressing the rat metabotropic glutamate receptor 5 (mGlu5) at concentrations in the higher ranges of the mGlu5 IC50's described in table 14.

### Effect of photoisomerization on mGlu5 responses

### Materials and methods

Cell culture and transient transfection single-cell calcium imaging, solutions and reagents, were as explained above for the mGlu5 response experiments.

*Photoisomerization:* Photoisomerization of the photochromic compounds was achieved by illuminating the specimen between each Fura-2 fluorescence measurement (i.e. calcium imaging, as explained above) with light flashes of high intensity and 0.5 Hz pulse-repetition frequency of 490 nm wavelength (0.1 s duration), and 390 nm (0.1 to 1 s duration) achieved by the Flash Tool of the Protocol Editor within the TILLvisION imaging software. The light source and the filter used for image acquisition were the same as for the photoisomerization step.

### Activity results

We explored the photoregulation of some compounds on HEK tsA201 cells transiently expressing the rat metabotropic glutamate receptor 5 (mGlu5) by single-cell calcium imaging. First, receptor activation was triggered by adding 3 µM quisqualate into the recording chamber. Secondly, compounds were added, still in the presence of the agonist, and monitored how the different molecules influenced the cell response. Finally, photoismerization was performed.

The activity of SIB-1757, **47** and **1c** was studied. The *cis-* isoforms of these molecules were obtained by illuminating SIB-1757, **((*E*)-(47))** at 380 or **(*E*)-1c** at 390 nm. The *trans-* configurations were restored under 500 and 490 nm light, respectively.

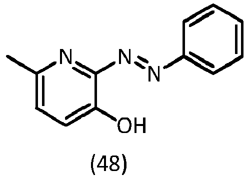

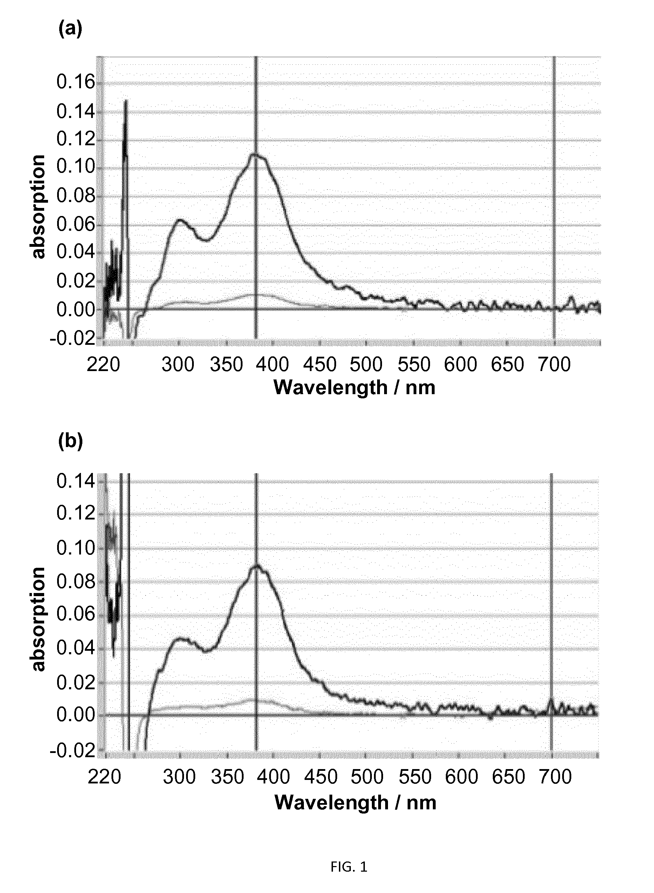

**SIB-1757 (48)** (Sigma Aldrich) is a moderate-affinity non-competitive antagonist of mGlu5 [J. Pharmacol. Exp. Thr., 1999, 290, 170-181]. This molecule contains an azobenzene group, but the UV-VIS absorption spectrum did not reveal any photoswitch following irradiation of SIB-1757 **(48)** with ultraviolet light (Figure 1). However, we tested the molecule on a cell line heterogously expressing mGlu5, to ensure that the biological effect of SIB-1757 **(48)** on the target receptor is not light dependent. The results obtained show that the molecule is clearly an antagonist of mGlu5, as previously described (it can revert the cellular response to the agonist quisqualate). Also, the response to the agonist and in the presence of the molecule does not change when we shine light on the cell (dotted box: UV light; lined box: visible light). This demonstrates that the activity of SIB-1757 **(48)** is not light-dependent despite the presence of the azobenzene group (see Figure 2). Moreover, we observed a high lipophilicity of the molecule, and also a low solubility, which make SIB-1757 **(48)** quite uneasy to handle for biological applications (not shown).

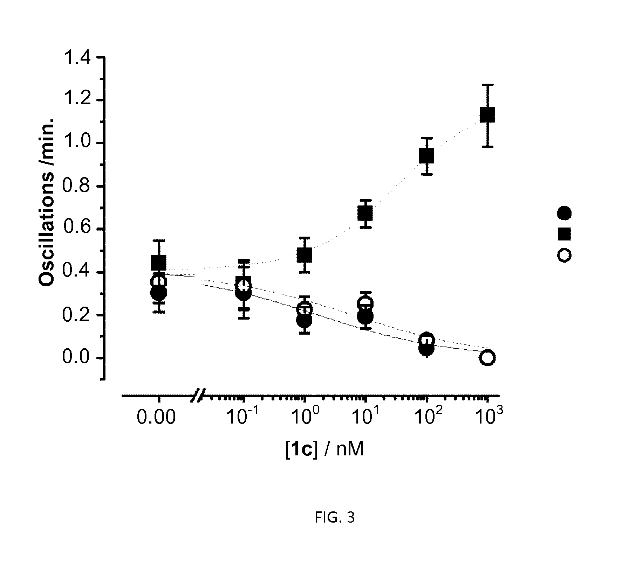

We characterized in detail the effect of compound **1c** on mGlu5 activation, using calcium imaging of mammalian cells exogenously expressing the receptor. Light pulses required for calcium imaging can be combined with dark intervals, or with illumination at 390 nm or 490 nm to photoisomerize the compounds in the field of view [Proc. Natl. Acad. Sci. USA, 2007, 104 (26), 10865-10870]. Stimulation of mGlu5 with orthosteric agonists activates phospholipase C and results in oscillatory intracellular calcium responses [Nature, 1996, 383(6595), 89-92] that can be quantified in individual cells [J Biol. Chem., 2002, 277(39), 35947-35960; J. Biol. Chem., 2005, 280(27), 25409-25415] and have a frequency about 1 oscillation/min *[*Mol. Pharm., 2009, 76(6), 1302-1313]. We observed that in the dark or under 490 nm light, oscillatory responses to 3 µM quisqualate are slowed down in a concentration-dependent manner by compound **1c,** and were completely blocked at 100 nM (Figure 2). However, calcium oscillations were immediately resumed upon 390 nm illumination, and could be reversibly switched on and off at 390 nm and 490 nm (Figure 2). At 100 nM, a saturating inhibitory concentration in dark conditions according to Figure 2, the oscillatory frequency at 390 nm was significantly higher than with quisqualate alone. Full dose-response curves in the dark and under 490 nm and 390 nm illumination, were obtained from calcium responses to quisqualate and compound **1c** at different concentrations (Figure 3). The inhibitory profile in the dark or under 490 nm light corresponds to an antagonistic behaviour with IC₅₀ = 30 nM, and cellular responses are similar to those of MPEP, a selective mGlu5 antagonist [Neuropharm., 1999, 38(10), 1493-1503].

The increase in calcium oscillation frequency of compound **1c** under 390 nm light can be associated to an enhancement of the activity of the receptors due to the light promoted isomerization of the compound **1c.** Overall, these results identify compound **1c** as a photoswitchable allosteric modulator.

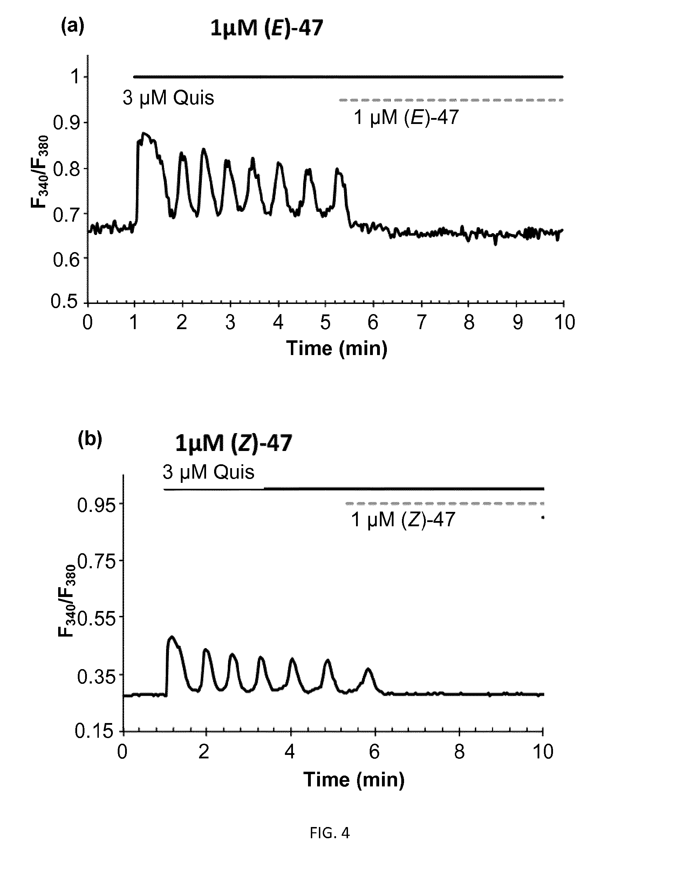

Compound **(E)-47** is an analogous of the **(*E*)-1c** in which the nitrogen atoms in the azobenzene group were substituted by carbon atoms. As could be expected in simple terms, **(E)-47** should be an antagonist of mGlu5 because is similar in geometric and chemical terms to **(*E*)-1c,** which was confirmed as it also blocked the oscillatory responses of quisqualate like compound **1c** did. (Figure 4).

Compound **(Z)-47** is an analogous of the compound **(*Z*)-1c** in which the nitrogen atoms in the azobenzene group have been substituted by carbon atoms. This molecule allowed us to test whether a rigid and similar *cis* configuration of the molecule defines the same pharmacological profile of **(*Z*)-1c,** which has been previously associated to the enhancement of the mGlu5 response. In other words, we tested if the *cis* geometry was sufficient to determine its potentiation of mGlu5 response. Our results show (see Figure 4) not only that **(Z)-47** did not potentiate mGlu5 responses as it could be expected in simple terms, but also they evidence that **(Z)-47** inhibits mGlu5 activation. It seems therefore that **(Z)-47,** like **(E)-47** and the original molecule **(*E*)-1c,** is an mGlu5 antagonist.

Compounds *cis* and *trans* **47** are C=C analogues of the *cis* and *trans* form of the azobenzene **1c,** respectively. Both **(*E*)-47** and **(*E*)-1c** inhibit mGlu5 response, but whereas **(*Z*)-47** shows also an inhibition of the receptor response, **(*Z*)-1c** increases this response. As a conclusion, we show that the geometrical correlation of molecular structure (N=N or C=C) does not involve a correspondence in its biological activity.

## Claims

1. A compound of formula (1): 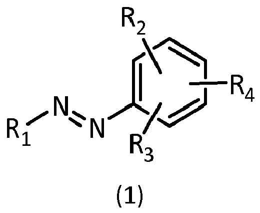 wherein
R₁ is selected from 2-pyridyl and 3-pyridyl, optionally substituted at the carbon atoms with one, two or three substituents independently selected from the group consisting of -halogen, -OH, -OR₅, -OCF₃, -SR₅, -NR₅R₆, -NHCOR₅, -COOR₅, -OCOR₅,-NO₂, -COR₅, -CONR₅R₆, -SO₂NH_{2,} (C₁-C₆) alkyl and (C₆-C₁₀) aryl;
R₂ and R₃ are independently selected from -H, halogen, -OR₅, -OCF₃, -SR₅, -NR₅R₆, -NHCOR₅, -COOR₅, -OCOR₅, -NO₂, -CN, -COR₅, -CONR₅R₆, -SO₂NH₂, -NHSO₂R₅, (C₁-C₆) alkyl, and (C₆-C₁₀) aryl;
R₄ is selected from -NR₇CO-R₈, -CONR₇-R₉, -NHSO₂-R₉, -S-R₉, -O-R₉, -NR₇CONH-R₁₀ and -N-phtalimidyl group;
R₅ and R₆ are independently selected from H, (C₁-C₆) alkyl and (C₆-C₁₀) aryl;
R₇ is selected from H, -CO-(C₁-C₅) alkyl, -CO-(C₆-C₁₀) aryl, (C₄-C₆) alkyl, (C₆-C₁₀) aryl and heterocyclyl; wherein the heterocyclyl group is linked to the -CON- or -NCO- moieties by a ring carbon atom;
R₈ is selected from (C₃-C₈) alkyl; (C₃-C₈) cycloalkyl; (C₆-C₁₀) aryl and o heterocyclyl; wherein the heterocyclyl group is linked to the -NR₇CO- moiety by a ring carbon atom;
R₉ is selected from (C₁-C₈) alkyl; (C₃-C₈) cycloalkyl; (C₆-C₁₀) aryl and heterocyclyl; wherein the heterocyclyl group is linked to the -CON-, -NHSO₂-, -S-, or -O- moieties by a ring carbon atom;
R₁₀ is selected from (C₁-C₈) alkyl, (C₃-C₈) cycloalkyl, and (C₆-C₁₀) aryl;
wherein the alkyl, aryl, cycloalkyl and heterocyclyl moieties in R₂-R₃ and R₅-R₁₀ defined above are optionally substituted, with one, two or three substituents independently selected from the group consisting of -halogen, -OH, -OR₅, -OCF₃,-SR₅, -NR₅R₆, -NHCOR₅, -COOR₅, -OCOR₅, -NO₂, -COR₅, -CONR₅R₆, -SO₂NH₂, (C₁-C₆) alkyl and (C₆-C₁₀) aryl;
R₄ is in *meta* or *para* position in respect to the R₁-N=N- moiety; and
provided that the compound of formula (1) contains from 2 to 4 ring systems, only 1 -N=N- group and that the compound of formula (1) is not 2-amino-N-[4-[2-(3-pyridinyl)diazenyl]phenyl]benzamide;
or a pharmaceutically acceptable salt, stereoisomer or solvate thereof.

2. Compound of formula (1) according to claim 1, wherein R₁ is unsubstituted 2-pyridyl.

3. Compound of formula (1) according to any preceding claim, wherein R₂ and R₃ are independently selected from -H, -OR₅, and -CN.

4. Compound of formula (1) according to claim 3, wherein one of R₂ and R₃ is -OR₅ and wherein R₅ is selected from unsubstituted (C₁-C₆) alkyl.

5. Compound of formula (1) according to any of claims 3 or 4, wherein one of R₂ and R₃ is H.

6. Compound of formula (1) according to claim 5, wherein R₂ is H and R₃ is in *meta* position in respect to the R₁-N=N- moiety.

7. Compound of formula (1) according to any preceding claim, wherein R₄ is selected from -NR₇CO-R₈, -CONR₇R₉, -NR₇CONH-R₁₀ and phthalimidyl group.

8. Compound of formula (1) according to claim 7, wherein R₄ is -NR₇CO-R₈, wherein R₇ is H or phenyl optionally substituted with CN.

9. Compound of formula (1) according to any preceding claim, wherein R₈ is selected from unsubstituted (C₃-C₈) alkyl; unsubstituted (C₃-C₈) cycloalkyl; phenyl optionally substituted with 1, 2 or 3 substituents independently selected from -CO-phenyl, halogen, and -CN; pyridyl; and tiazolyl.

10. Compound of formula (1) according to claim 9, wherein R₈ is selected from unsubstituted (C₃-C₅) alkyl; unsubstituted (C₅-C₇) cycloalkyl; phenyl substituted with 1 or 2 substituents independently selected from halogen, preferably Cl; and pyridyl.

11. Compound of formula (1) according to any preceding claim, wherein R₄ is in para position in respect to the R₁-N=N- moiety.

12. Compound of formula (1) according to claim 1 selected from the group consisting of:
- (E)-4-benzoyl-N-(2-methoxy-4-(pyridin-2-yldiazenyl)phenyl)benzamide;
- (Z)-4-benzoyl-*N*-(2-methoxy-4-(pyridin-2-yldiazenyl)phenyl)benzamide;
- (E)-2,4-dichloro-N-(2-methoxy-4-(pyridin-2-yldiazenyl)phenyl)benzamide;
- (Z)-2,4-dichloro-N-(2-methoxy-4-(pyridin-2-yldiazenyl)phenyl)benzamide;
- (E)-2-chloro-N-(2-methoxy-4-(pyridin-2-yldiazenyl)phenyl)benzamide;
- (Z)-2-chloro-N-(2-methoxy-4-(pyridin-2-yldiazenyl)phenyl)benzamide;
- (E)-N-(2-methoxy-4-(pyridin-2-yldiazenyl)phenyl)picolinamide;
- (Z)-N-(2-methoxy-4-(pyridin-2-yldiazenyl)phenyl)picolinamide;
- (E)-N-(2-methoxy-4-(pyridin-2-yldiazenyl)phenyl)nicotinamide;
- (Z)-N-(2-methoxy-4-(pyridin-2-yldiazenyl)phenyl)nicotinamide;
- (E)-N-(2-methoxy-4-(pyridin-2-yldiazenyl)phenyl)isonicotinamide;
- (Z)-N-(2-methoxy-4-(pyridin-2-yldiazenyl)phenyl)isonicotinamide;
- (E)-N-(2-cyano-4-(pyridin-2-yldiazenyl)phenyl)picolinamide;
- (Z)-N-(2-cyano-4-(pyridin-2-yldiazenyl)phenyl)picolinamide;
- (E)-N-(4-(pyridin-2-yldiazenyl)phenyl)picolinamide;
- (Z)-N-(4-(pyridin-2-yldiazenyl)phenyl)picolinamide;
- (E)-N-(3-methoxy-4-(pyridin-2-yldiazenyl)phenyl)picolinamide;
- (Z)-N-(3-methoxy-4-(pyridin-2-yldiazenyl)phenyl)picolinamide;
- (E)-2-(2-methoxy-4-(pyridin-2-yldiazenyl)phenyl)-isoindoline-1,3-dione;
- (Z)-2-(2-methoxy-4-(pyridin-2-yldiazenyl)phenyl)-isoindoline-1,3-dione;
- (E)-N-benzoyl-N-(2-methoxy-4-(pyridin-2-yldiazenyl)phenyl)benzamide;
- (Z)-N-benzoyl-N-(2-methoxy-4-(pyridin-2-yldiazenyl)phenyl)benzamide;
- (E)-N-(2-methoxy-4-(pyridin-2-yldiazenyl)phenyl)benzamide;
- (Z)-N-(2-methoxy-4-(pyridin-2-yldiazenyl)phenyl)benzamide;
- (E)-N-(2-methoxy-4-(pyridin-2-yldiazenyl)phenyl)isobutyramide;
- (Z)-N-(2-methoxy-4-(pyridin-2-yldiazenyl)phenyl)isobutyramide;
- (E)-N-(2-methoxy-4-(pyridin-2-yldiazenyl)phenyl)cyclohexanecarboxamide;
- (Z)-N-(2-methoxy-4-(pyridin-2-yldiazenyl)phenyl)cyclohexanecarboxamide;
- (E)-1-(2-fluorophenyl)-3-(2-methoxy-4-(pyridin-2-yldiazenyl)phenyl)urea;
- (Z)-1-(2-fluorophenyl)-3-(2-methoxy-4-(pyridin-2-yldiazenyl)phenyl)urea;
- (E)-2-fluoro-N-(2-methoxy-4-(pyridin-2-yldiazenyl)phenyl)benzamide;
- (Z)-2-fluoro-N-(2-methoxy-4-(pyridin-2-yldiazenyl)phenyl)benzamide;
- (E)-2-cyano-N-(2-cyanobenzoyl)-N-(2-methoxy-4-(pyridin-2-yldiazenyl)phenyl)benzamide;
- (Z)-2-cyano-N-(2-cyanobenzoyl)-N-(2-methoxy-4-(pyridin-2-yldiazenyl)phenyl)benzamide;
- (E)-N-(2-methoxy-4-(pyridin-2-yldiazenyl)phenyl)-1,3-thiazole-4-carboxamide;
- (Z)-N-(2-methoxy-4-(pyridin-2-yldiazenyl)phenyl)-1,3-thiazole-4-carboxamide;
- (E)-N-(pyridin-2-yl)-4-(pyridin-2-yldiazenyl)benzamide;
- (Z)-N-(pyridin-2-yl)-4-(pyridin-2-yldiazenyl)benzamide;
- (E)-N-(3-(pyridin-2-yldiazenyl)phenyl)picolinamide; and
- (Z)-N-(3-(pyridin-2-yldiazenyl)phenyl)picolinamide;
or a pharmaceutically acceptable salt, stereoisomer or solvate thereof.

13. Compound of formula (1) as defined in any preceding claim, or a pharmaceutically acceptable salt, stereoisomer or solvate thereof, for use as a medicament.

14. Compound of formula (1) as defined in any of claims 1 to 12, or a pharmaceutically acceptable salt, stereoisomer or solvate thereof, for use in the treatment and/or prevention of a disease or condition selected from the group consisting of psychosis, epilepsy, schizophrenia, Alzheimer's disease, cognitive disorders, memory deficits, Parkinson's disease, hypoxia, ischemia, dementia caused by AIDS, migraine, depression, mood disorders, anxiety disorders, fragile X syndrome, pain syndromes, drug or alcohol abuse or addiction, substance tolerance or dependence, and drug withdrawal, bulimia nervosa, anorexia nervosa, gambling dependence, smoking, sex dependence obsessive compulsive disorders, stomach diseases and Ophelia syndrome.

15. Pharmaceutical composition comprising a compound of formula (1) as defined in any of claims 1 to 12, or a pharmaceutically acceptable salt, stereoisomer or solvate thereof, and a pharmaceutically acceptable excipient.
